# EUROPÄISCHE PATENTSCHRIFT

(11) **EP 0 661 269 B1**
(45) Veröffentlichungstag und Bekanntmachung des Hinweises auf die Patenterteilung: **26.03.1997**
(21) Anmeldenummer: 94120766.4
(22) Anmeldetag: 27.12.1994
(51) Int. Cl.: C07D 213/81, C07D 213/82, C07D 213/89, C07D 215/48, A61K 31/44, A61K 31/47

(54) **Substituierte heterocyclische Carbonsäureamide, ihre Herstellung und ihre Verwendung als Arzneimittel**
Substituted heterocyclic carboxylic acid amides, their preparation and their use as medicaments
Amides d'acide carboxylique hétérocycliques substitués leur préparation et leur utilisation comme médicaments

(30) Priorität: 30.12.1993 DE 4344958; 09.11.1994 DE 4439935
(43) Veröffentlichungstag der Anmeldung: 05.07.1995
(73) Patentinhaber: HOECHST AKTIENGESELLSCHAFT, 65929 Frankfurt am Main (DE)
(72) Erfinder: Weidmann, Klaus, Dr., D-61476 Kronberg (DE); Baringhaus, Karl-Heinz, Dr., D-61200 Wölfersheim (DE); Tschank, Georg, Dr., D-55270 Klein-Winternheim (DE); Bickel, Martin, Dr., D-61348 Bad Homburg (DE)

(56) Entgegenhaltungen:
- EP-A- 0 562 512
- BULLETIN DE LA SOCIETE CHIMIQUE DE FRANCE, Nr.8, 1965, PARIS FR Seiten 2252 - 2259 G. JOLLES ET AL. 'Pristinamycine. Synthèse de l'heptapeptide linéaire et d'oligopeptides correspondant au constituant Ia de la pristinamycine.'
- JOURNAL OF MEDICINAL CHEMISTRY, Bd.37, Nr.18, 2. September 1994, WASHINGTON US Seiten 2889 - 2895 R.J. BERGERON ET AL. 'An investigation of desferrithiocin metabolism'
- CHEMICAL ABSTRACTS, vol. 115, no. 21, 25. November 1991, Columbus, Ohio, US; abstract no. 222778s, T.J. FRANKLIN ET AL. 'Approaches to the design of anti-fibrotic drugs.' Seite 21 ;
- CHEMICAL ABSTRACTS, vol. 95, no. 17, 26. Oktober 1981, Columbus, Ohio, US; abstract no. 151135k, M. KONISHI ET AL. 'Structure determination of a new antitumor antibiotic, BBM-928.' Seite 720 ;
- Arzneim. Forsch. 41,No4,392-398(1991)
- Drugs Des. Delivery 6,No1,15-28(1990)

## Beschreibung

Die Erfindung betrifft substituierte heterocyclische Carbonsäureamide, ihre Herstellung und ihre Verwendung als Inhibitoren der Prolyl-4-hydroxylase und ihre Verwendung als Arzneimittel zur Behandlung von fibrotischen Erkrankungen

Verbindungen, die die Enzyme Prolin- und Lysinhydroxylase inhibieren, bewirken eine sehr selektive Hemmung der Kollagenbiosynthese durch Beeinflussung der kollagenspezifischen Hydroxylierungsreaktionen. In deren Verlauf wird proteingebundenes Prolin oder Lysin durch die Enzyme Prolin- bzw. Lysinhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma und Vernarbungen nach Verbrennungen, Verletzungen und chirurgischen Eingriffen) und sowie die Atherosklerose.

Es ist bekannt, daß das Enzym Prolinhydroxylase durch Pyridin-2,4- und -2,5-dicarbonsäure effektiv gehemmt wird (K. Majamaa er al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238 (1987) 625 bis 633). Auch Prodrugs der Pyridin-2,4(5)-dicarboxylate sind bekannt. Diese sind in den älteren deutschen Anmeldungen P 42 33 124.2, P 42 38 506.7 und P 42 09 424.0 beschrieben.

N-Oxalylglycine als Inhibitoren der Prolyl-4-hydroxylase sind aus J. Med. Chem. 1992, 35, 2652 bis 2658 (Cunliffe et al.), und EP-A-0 457 163 (Baader et al.) bekannt.

3-Hydroxypyridin-2-carbonsäure-N-(carboxymethyl)amid ist aus G. Yolles et al. in: Bull. Soc. Chim Fr. 1965, 8, 2252 bis 2259 bekannt.

Hydroxyisochinoline- und Hydroxycinnolincarbonsäureglycylamide sind aus Biochem. Soc. Trans. 1991, 19, 812 bis 815 (Franklin et al.) bekannt.

Überraschenderweise wurde nun gefunden, daß heterocyclische Carbonsäureamide mit einer OH- oder SH-Funktion in ortho-Position zur Amidfunktion stark wirksame Inhibitoren der Prolyl-4-hydroxylase sind.

Die erfindungsgemäßen Verbindungen entsprechen der allgemeinen Formel I 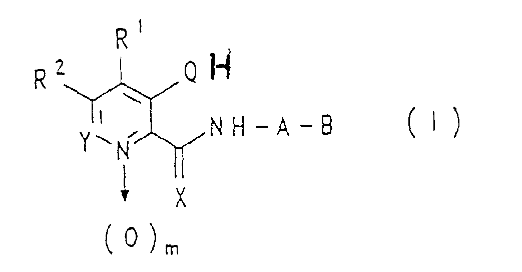 in welcher
Q O oder S,
X O,
Y C_{R}3,
m 0 und 1,
A (C₁-C₃)-Alkylen, das gegebenenfalls einfach substituiert ist mit Halogen, Cyano, Trifluormethyl, (C₁-Cₑ)-Alkyl, (C₁-Cₑ)-Hydroxyalkyl, (C₁-Cₑ)-Alkoxy, -O-[CH₂]ₓ C_{f}H(2f+1-g)F_{g} oder
A -CHR⁵-bedeutet, wobei R⁵ einen der Substituenten des a-C-Atoms einer a-Aminosäure bedeutet, insbesondere einer natürlichen L-Aminosäure und ihres D-isomeren, wobei R⁵ nicht -CH₂SH bedeutet,
B C0₂H,
R² Wasserstoff, (_{C}1-C₂₀)-Alkyl, (C₂-C₂ₒ)Alkenyl, (C₂-C₂₀)-Alkinyl, (C₁-C₂ₒ)-Alkoxy, (C₁-C₂₀)-Alkenyloxy, (C₂-C₂ₒ)-AI- klnyloxy, Retinyloxy, (C₁-C₂₀)-Alkoxy-(C₁-C₃)-alkyl, (C₂-C₂₀)-Alkenyloxy-(C₁-C₃)-alkyl, Retinyloxy-(C₁-C₃)-alkyl, (C₂₋C₂ₒ)-Alkinyloxy-(C₁-C₃)-alkyl, Halogen, Cyano, Trifluormethyl, (C₁-C₈-Hydroxyalkyl, (C₁-C₂₀)-Alkanoyl,(C₇-C₁₆)-Aralkanoyl, (C₆-C₁₂)-Aroyl, (C₆-C₁₂)-Aryl, (C₇-C₁₆)-Aralkyl, -O-[CH₂)ₓC_{f}H(2f+1-g)F_{g}, NR'R", (C₁-C₁₀)-Alkylmercapto, (C₁-C₁₀)-Alkylsulfinyl, (C₁-C₁₀)-Alkylsulfonyl, (C₆-C₁₂)-Arylmercapto, (C₆-C₁₂)-Arylsulfinyl), (C₆-C₁₂)-Arylsulfonyl, (C₇-C₁₂)-Aralkylmercapto, (C₇-C₁₂)-Aralkylsulfinyl, (C₇-C₁₂)-Aralkylsulfonyl, (C₆-C₁₂)-Aryloxy, (C₇-C₁₆)-Aralkyloxy, Carboxy, (C₁-C₂ₒ)-Alkoxycarbonyl, (C₁-C₁₂)-Alkoxy-(C₁-C₁₂)-alkoxycarbonyl, (C₆-C₁₂)-Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂ₒ)-Alkinyloxycarbonyl, (C₃-C₈)-Cycloalkyl-(C₁-C₆)-alkoxycarbonyl, (C₃-C₈)-Cycloalkoxy-(C₁-C₆)-alkoxycarbonyl, (C₆-C₁₂)-Aryloxy-(C₁-C₆)-alkoxycarbonyl, (C₇-C₁₆)-Aralkoxy-(C₁-C₆)-alkoxycarbonyl, Carbamoyl, N-(C₁-C₁₂)-Alkylcarbamoyl; N, N-Di-(C₁-C₁₂)-alkylcarbamoyl, N-(C₃-C₈)-Cycloalkylcarbamoyl, N,N-Di- cyclo(C₃-C₈)-alkylcarbamoyl, N-(C₁-C₁₀)-Alkyl-N-(C₃-C₈)-cycloalkylcarbamoyl, N-(C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroabietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₇-C₁₆)-Alkyl-N-(C₇-C₁₆)-aralkylcarbamoyl, N-((C₁-C₁₂)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₇-C₁₆)-alkyl) carbamoyl;N-(C₁-C₁₀)-Alkyl-N-((C₁-C₁₀)-alkoxy-(C₁-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann bedeutet,
   wobei Aryl in der Weise substiuiert ist wie für R¹ und R³ definiert,
R¹ und R³ gleich oder verschieden sind und Wasserstoff, Halogen, (C₁-C₁₂)-Alkyl, (C₁-C₁₂)-Alkoxy, -O-(CH₂)ₓ-C_{f}H_{(2f+1-g)}Hal_{g}, (C₁-C₈)-Alkoxy-(C₁-C₁₂)-alkyl, (C₁-C₈)-Alkoxy-(C₁-C₁₂)-alk- oxy, (C₁-C₁₂)-Alkoxy-(C₁-C₈)-alkoxy-(C₂-C₆,)-alkyl, (C₇-C₁₁)-Aralkyloxy, (C₃-C₈)-Cycloalkyl, (C₃-C₈)-Cycloalkyl-(C₁-C₈)-alkyl, (C₃-C₈)-Cycloalkyloxy, (C₃₋C₈)-Cycloalkyl-(C₁-C₈)-alkoxy, (C₃-C₈)-CyCloalkyloxy-(C₁-C₈)-alkyl, (C₃-C₈)-Cycloalkyloxy-(C₁-C₈)-alkoxy, (C₃-C₈)-Cycloalkyl-(C₁-C₆)-alkyl-(C₁-C₆)-alkoxy, (C₃-C₈)-Cycloalkyl-(C₁-C₆)-alkoxy-(C₁-C₆)-alkyl, (C₃-C₈)-CyCloalkoxy-(C₁-C_{6;})-alkoxy-(C₁-C₆)-alkyl, NR^{Y}R^{Z}, (C₁-C₈)-Alkylmorcapto, (C₁-C₈)-Alkylsulfinyl oder (C₁-C₈)-Alkylsulfonyl, (C₆-C₁₂)-Arylmercapto, (C₆-C₁₂)-Arylsulfi- nyl, (C₆-C₁₂)-Arylsulfonyl, (C₇-C₁₂)-Aralkylmercapto, (C₇-C₁₁)-Aralkylsulfinyl, (C₇-C₁₁)-Aralkylsulfonyl, substituiertes (C₆-C₁₂)-Aryloxy-(C₁-C₆)-alkyl, (C₇-C₁₁)-Aralkoxy-(C₁-C₆)-alkyl, (C₆-C₁₂)-Aryloxy-(C₁-C₆)-alkoxy-(C₁-C₆)-alkyl, (C₇-C₁₁)-Aralkyloxy-(C₁-C₆)-alkoxy-(C₁-C₆)-alkyl, (C₆-C₁₂)-Aryloxy, (C₇-C₁₁)-Aralkyloxy, (C₆ C₁₂)-Aryloxy-(C₁-C₆)-alkoxyoder (C₇-C₁₁)-Aralkoxy-(C₁-C₆)-alkoxy bedeutet, wobei ein aromatischer Rest mit 1, 2, 3, 4 oder 5 gleichen oder verschiedenen Substituenten aus der Reihe Wasserstoff, Halogen, Cyano, Nitro, Trifluormethyl, (C₁-C₁₆)-Alkyl,(C₁-C₁₆)-Alkenyl, (C₁-C₆)-Hydroxyalkyl, (C₁-C₁₆)-Alk- oxy, (C₁-C₁₆)-Alkenyloxy; -O-[CH₂]ₓC_{f}H_{(2f+1-g)}F_{g}, -OCF₂Cl, -O-CF₂-CHFCI, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy, (C₃-C₈)-Cycloalkylcarbamoyl, Phenyl, Benzyl, Phenoxy, Benzyloxy, NR^{Y}R^{Z}, Phenylmercapto, Phenylsulfonyl, Phenylsulfinyl, Sulfamoyl, N-(C₁-C₄)-AI- kylsulfamoyl oder N,N-Di-(C₁-C₄)-alkylsulfamoyl trägt, oder gegebenenfalls bis zu 3 der vorstehend genannten gleichen oder verschiedenen Substituenten trägt und zwei benachbarte C-Atome des Aralkyloxyrestes gemeinsam eine Kette -[CH₂-] und/oder -CH=C-CH= CH- tragen, wobei eine CH₂-Gruppe der Kette gegebenenfalls durch 0, S, SO, S0₂ oder NR' ersetzt ist,
R² und R³ zusammen mit dem sie tragenden Pyridin einen Chinolin-Ring der Formel 1 a bilden, wobei
R¹³, R¹⁴, R¹⁵ und R¹⁶ Wasserstoff, (C₁-C₁₂)-Alkyl, (C₁-C₁₂)-Alkenyl, Chlor, Fluor, Brom, Trifluormethyl, (C₁-C₁₂)-Alkylsulfonyl, (C₁-C₁₂)-Alkylsulfinyl, Phenylsulfonyl, Phenylsulfinyl; wobei Phenyl gegebenenfalls mit Fluor, Chlor oder (C₁-C₅)-Alkoxy substituiert ist, (C₁-C_{1O})-Alkoxy, -O-[CH₂]ₓ-C_{f}H(_{2f+1-g}), Fg oder einen Rest der Formel D bedeutet 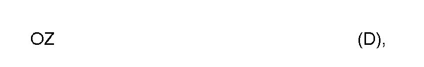
wobei Z für [CH₂]ᵥ- [O]_{w}-[CH₂]ₜ-E steht,
worin E einen substituierten Phenylrest der Formel F 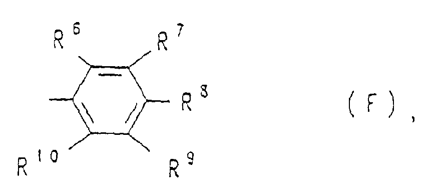 oder einen substituierten Heteroaryl-Rest oder einen substituierten (C₃-C₈)-Cycloalkylrest bedeutet,
   wobei
   v = 0, 1, 2, 3, 4, 5, 6 w = 0,1 und t = 0, 1, 2, 3, mit der Einschränkung, daß v ungleich 0 ist, falls w= 1 ist bedeutet und R⁶, R⁷, R⁸, R⁹ und R¹⁰ gleich oder verschieden sind und Wasserstoff, Halogen, Cyano, Nitro, Trifluormethyl, (C₁-C₆)-Alkyl; (C₃-C₈-Cycloalkyl, (C₁-C₆)-Alkoxy, -O-[CH₂₋]ₓC_{f}H_{(2f+1-g)}F_{g}, -OCF₂CI, -O-CF₂-CHFCI, (C₁-C₆)-Alkylmercapto, (C₁-C₆)-Hydroxyalkyl, (C₁-C₆)-Alkoxy-(C₁-C₆)-alkoxy, (C₁-C₆)-Alkoxy-(C₁-C₆,)-alkyl, (C₁-C₆)-Alkylsultinyl,(C₁-C₆)-Alkylsulfonyl, (C₁-C₆)-Alkylcarbonyl, (C₁-C₈)-Alkoxycarbonyl,Carbamoyl, N-(C₁-C₈)-Alkylcarbamoyl, N, N-Di-(C₁-C₈)-alkylcarbamoyl, gegebenenfalls mit Fluor, Chlor, Brom, Trifluormethyl und (C₁-C₆)-Alkoxy-substituiertes (C₇-C₁₁)-Aral- kylcarbamoyl, N-(C₃-C₈)-Cycloalkylcarbamoyl, N-(C₃-C₈)-Cycloalkyl-(C₁-C₄)-alkylcarbamoyl, (C₁-C₆)-Alkylcarbonylo- xy, Phenyl, Benzyl, Phenoxy, Benzyloxy, NR'R", wie Amino, Anilino, N-Methylanilino, Phenylmercapto, Phenylsulfonyl, Phenylsulfinyl, Sulfamoyl, N-(C₁-C₈)-Alkylsulfamoyl oder N,N-Di-(C₁-C₈)-alkylsulfamoyl bedeuten, oder zwei benachbarte Substituenten gemeinsam eine Kette -[CH₂-]ₙ oder -CH=CH-CH=CH- bedeuten, wobei eine CH₂-Gruppe der Kette gegebenenfalls durch O, S, SO, S0₂ oder NR' ersetzt ist,
falls R¹ und/oder R³ in der Bedeutung von (C₆-C₁₂)-Aryloxy, (C₇C₁₁ )-Aralkyloxy, (C₆-C₁₂)-Aryloxy-(C₁-C₆)-alkoxy, (C₇-C₁₁)-Aralkoxy-(C₁₋C₆)-alkoxyoder einem entsprechenden endständige Cycloalkyl-Gruppen enthaltenden Rest stehen, so bedeutet dieser Rest vorzugsweise einen Rest der Formel D 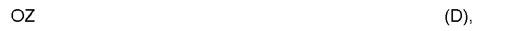
wobei Z für [CH₂]ᵥ- [O]_{w}-[CH_{2]t}-E steht,
falls R¹ und/oder R³ in der Bedeutung von (C₇-C₁₁)-Aralkyl,
(C₆-C₁₂)-Aryloxy-(C₁-C₆)-alkyl, (C₇-C₁₁)-Aralkoxy-(C₁-C₆)-alkyl oder einem entsprechenden endständige Cycloalkyl-Gruppen enthaltenden Rest stehen, so bedeutet dieser Rest vorzugsweise einen Rest der Formel Z, wobei Z in der obigen Bedeutung steht, aber v # 0 ist, und
R^{Y} und R^{Z} gleich oder verschieden sind und Wasserstoff, (C₆-C₁₂)-Aryl, (C₁-C₁₀)-Alkyl, (C₃-C₁₀)-Cycloalkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl, (C₁-C₁₀)-Al- kanoyl, ggf. substituiertes (C₇-C₁₆,)-Aralkanoyl, ggf. substituiertes (C₆-C₁₂)-Aroyl bedeuten, oder
R^{Y} und R^{Z} gemeinsam für -[CH_{2]h-} stehen, worin eine CH₂-Gruppe durch O, S, N-(C₁-C₄)-Alkanoylimino oder N-(C₁-C₄)-Alkoxycarbonylimino ersetzt sein kann, und
f 1 bis 8,
g 0, 1 bis (2f+1
h 3 bis 6,
x 0 bis 3, und
n 3 oder 4 ist,

einschließlich der physiologisch wirksamen Salze, ausgenommen N-(3-Hydroxypicolinyl)-threonin, N-[(3-Hydroxy-6-methoxy-2-chinolinyl)-carbonyl]-D-serin, ist.

Unter Aryl wird insbesondere Phenyl und Napthyl, unter Heteroaryl insbesondere Pyridyl, Picolyl oder Thienylmethyl, unter Cycloalkyl vorzugsweise Cyclohexyl und unter Halogen insbesondere Fluor, Chlor und Brom verstanden.

Die Erfindung umfaßt weiterhin Salze der Verbindungen der allgemeinen Formel I.

Die Salzbildung mit basischen Reagenzien kann ein- oder zweifach an den aciden Gruppen der Verbindungen der Formel I, d.h. an den Resten B, R¹ , R², R³ und/oder an der aciden phenolischen OH(SH)-Gruppe, insbesondere an den Resten B, R² und der phenolischen OH(SH)-Gruppe erfolgen.

Zur Anwendung kommende Reagenzien sind beispielsweise Alkoholate, Hydroxide, Carbonate, Hydrogencarbonate, Hydrogenphosphate, Metallorganyle der Alkali- und Erdalkalielemente, der Elemente der 3. und 4. Hauptgruppe des Periodensystems und der Elemente der Übergangsmetalle,

Amine, gegebenenfalls 1- bis 3-fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches 1-bis 3-fach substituiert sein kann mit Hydroxy ocer (C₁-C₄)-Alkoxy, beispielsweise Tromethan, (Tris-Puffer), 2-Aminoethanol, 3-Aminopropanol, Hydroxylamin, Dimetrylhydroxylamin, 2-Methoxy-ethylamin, 3-Ethoxypropylamin, und basische Aminosäuren und -derivate, wie Aminosäureester, Histidin, Arginin und Lysin und deren Derivate, sowie

Arzneimitiel, die eine basische Gruppe enthalten, wie beispielsweise ®Amilorid, ®Verapamil und Betablocker.

Die Erfindung betrifft weiterhin die Verbindungen, gemäß Formel zuzüglich 3-Hydroxypyridin-2-carbonsäure-N-(carboxymethyl)amid_{;} N-(3-Hydroxypicolinyl)-L-threonin und N-[(3-Hydroxy-6-methoxy-2-chinolinyl)-carbonyl]-D-serin zur Anwendung als Arzheimittel.

Besonders bevorzugt sind Verbindungen der Formel 1, in der
Q O oder S,
X O,
Y C_{R}3
m O,
A eine (C₁-C₂)-Alkylengruppe,
B CO₂H,
R² Wasserstoff, Brom, Chlor, Cyano, (C₁-C₁₈)-Alkyl, (C₁-C₈)-Alkoxy, (C₁-C₁₈)-Alkoxymethyl, (C₂-C₁ₛ)-Alke- nyloxymethyl, (C₂-C₁₈)-Alkinyloxymothyl,Carbamoyl, N-(C₁-C₁₀)-Alkylcarbamoyl, N-((C₁-C₁₂)-Alkoxy-(C₁-C₄)-alkyl)carbamoyl, N, N-Di-(C₁-C₈)-alkylcarbamoyl, N-(C₃ C₈)-Cycloalkylcarbamoyl, N-(C₆-C₁₂)-Phenylcarbamoyl, N-(C₇-C₁₂)-Phenylalkylcarbamoyl, N-(C₁-C₆)-Alkyl-N-(C₆-C₁₂)phenylcarbamoyl, N-(C₁-C₆)-Alkyl-N-(C₇C₁₂)-phenylalkylcarbamoyl, N-((C₁-C₆)-Alkoxy-(C₁-C₆)-alkyl)carbamoyl, Carboxy, (C₁-C₂₀)-Alkoxycarbonyl, (C₂-C₂₀)-Alkenyloxycarbonyl, Retinyloxycarbonyl, (C₃-C₈)-Cycloalkoxycarbo- nyl, (C₃-C₈)-Cycloalkyl-(C₁-C₆)-alkoxycarbonyl, (C₃-C₈)-Cycloalkoxy-(C₁-C₆)-alkoxycarbonyl, Phenyl-(C₁-C₆)-alkoxycarbonyl, Phonoxy-(C₁-C₆)-alkoxycarbonyl, Benzyloxy-(C₁-C₆,)-alkoxycarbonyl, wobei ein Phenylrest in der Weise substituiert ist wie für R¹ und R³ definiert und einer der Reste
R¹oder R³ Wasserstoff und der andere einen Rest aus der Reihe Wasserstoff, Fluor, Chlor, (C₁-C₈)-Alkyl, (C₁-C₁₀)-Alkoxy, (C₅-C₆)-Cycloalkyl, (C₅-C₆)-Cycloalkyl-(C₁-C₆)-alkyl, (C₅-C₆,)-Cycloalkyloxy, (C₅-C₆)-Cycloalkyl-(C₁-C₆)-alkoxy, (C₅-C₆)-Cycloalkyloxy-(C₁-C₆,)-alkyl, (C₁-C₆)-Cycloalkyloxy-(C₁-C₆)-alkoxy, (C₅-C₆)-Cycloalkyl-(C₁-C₄)-alkyl-(C₁-C₄)-alkoxy, (C₅-C₆)-Cycloalkyl-(C₁-C₄)-alkoxy-(C₁-C₂)-alkyl, (C₅-Cₛ)-Cycloalkoxy-(C₁-C₄)-alkoxy-(C₁-C₂)-alkyl, -O-(CH₂]ₓ₋C_{f}H_{(2f+1-g)}^{F}g, (C₁-C₈)-Alkoxy-(C₁-C₈)-alkyl, (C₁-C₆)-Alk- oxy-(C₁-C₆)-alkoxy, (C₁-C_{6;})-Alkoxy-(C₁-C₄)-alkoxy-(C₁-C₂)-alkyl, substituiertes (C₆-C₁₂)-Phonoxy, (C₇-C₁₁)-Phenylalkyloxy, (C₆-C₁₂)-Phenoxy-(C₁-C₆)-alkoxy oder (C₇-C₁₁)-Phenylalkoxy-(C₁-C₆)-alkoxy, Phenoxy-(C₁-C₄)-alkyl, (C₇-C₁₁)-Phenylalkyloxy-(C₁-C₄)-alkyl, Phenoxy-(C₁-C₄)-alkoxy-(C₁-C₂)-alkyl, (C₇-C₁₁)-Phonylalkyloxy-(C₁-C₄)-alkoxy-(C₁-C₂)-alkyl bedeutet, wobei ein aromatischer Rest mit 1, 2 oder 3 gleichen oder verschiedenen Substituenten aus der Reihe Fluor, Chlor, Cyano, Trifluormethyl, (C₁-C₁₂)-Alkyl, (C₂-C₁₂)-Alkenyl, (C₂-C₁₂)-Alkenyloxy, (C₁-C₁2)-Alkoxy, substituiert ist

und
falls R¹ oder R³ in der Bedeutung von (C₆-C₁₂)-Phenoxy, (C₇-C₁₁)-Phenylalkyloxy, (C₆-C₁₂)-Phenoxy-(C₁-C₆)-alkoxy, (C₇-C₁₁)-Phenylalkoxy-(C₁-C₆,)-alkoxy, (C₅-C₆)-Cycloalkyloxy, (C₅-C₆,)-Cycloalkyl-(C₁-C₆),alkoxy, (C₅-C₆,)-Cycloalkoxy-(C₁-C₆)-alkoxy oder (C₅-C₆)-Cycloalkyl-(C₁-C₄)-alkyl-(C₁-C₄)-alkoxy steht, dieser Rest im speziellen einen Rest der Formel D bedeutet 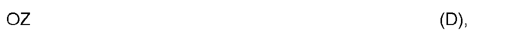
in der Z -[CH₂]ᵥ -[O]_{w} [CH₂]ₜ-E, bedeutet,
wobei E einen substituierten Phenylrest der Formel F 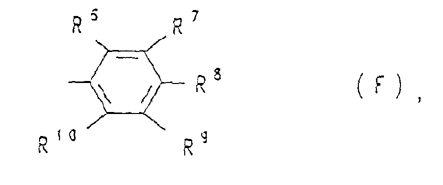
oder einen (C₃-C₈)-Cycloalkylrest bedeutet, wobei
v = 0, 1, 2, 3, w = 0, 1 und t = 0,1 sein kann, mit der Einschränkung, daß v ungleich 0 ist, falls w = 1 ist, und
worin R⁶, R⁷, R⁸, R⁹ und R¹⁰ gleich oder verschieden sind und Wasserstoff, Fluor, Chlor, Cyano, Trifluormethyl, (C₁-C₆)-Alkyl; (C₁-C₆)-Alkoxy, -O-[CH₂-]ₓC_{f}H_{(2f+1-g)}F_{g}, N-(C₁-C₈)-Alkylcarbamayl, N,N-Di-(C₁-C₈)-alkylcarbamoyl, N-(C₃-C₈)-Cycloalkylcarbamoyl, gegebenenfalls mit Fluor, Chlor, Trifluormethyl und (C₁-C₆)-Alkoxy substituiertes (C₇-C₁₁)-Phenylalkylcarbamoyl bedeuten, oder
falls R¹ oder R³ in der Bedeutung von Phenyl, Phenoxy-(C₁-C₆)-alkyl, (C₇-C₁₁)-Phenylalkyl (C₇-C₁₁)-Phenylalkyloxy-(C₁-C₄)-alkyl, (C₅-C₆)-Cycloalkyl, (C₅-C₆)-Cycloalkyl-(C₁-C₆)-alkyl, (_{C5}-C₆)-Cycloalkoxy-(C₁-C₄)-alkyl, (C₅-C₆)-Cycloalkyl-(C₁-C₄)-alkoxy-(C₁-C₂)-alkyl, (C₅-C₆)-Cycloalkoxy-(G₁-C₄)-alkoxy-(C₁-C₂)-alkyl, steht, dieser Rest im speziellen einen Rest der Formel Z bedeutet,
   worin
   v=1,2,3 und 4,w=0 und t = 0 ode
   v = 1,2, 3 und 4, w = 1 und t = 0 oder
   v =1,2,3 und 4,w =1,t =1

   sind.

Ganz besonders bevorzugt sind Verbindungen der Formel I, in der
Q O, S, bevorzugt O,
X O,
Y CR³
m O,
A eine -CH₂-Gruppe,
B -CO₂H,
_{R}1 Wasserstff,
R² und R³ zusammen mit dem sie tragenden Pyridin einen Chinolinring bilden, wobei
R¹³, R¹⁵ und R¹⁶ Wasserstoff,
R¹⁴ Wasserstoff, (C₁-C₁₂)-Alkyl, (C₁-C₁₂)-Alkenyl, Chlor, Fluor, Brom, Trifluormethyl, (C₁-C₁₂)-A1- kylsulfonyl, (C₁-C₁₂)-Alkylsulfinyl, Phenylsulfonyl, Phenylsulfinyl; wobei Phenyl gegebenenfalls einfach mit Fluor, Chlor oder (C₁-C₅)-Alkoxy substituiert ist, (C₁-C₁₀)-Alkoxy, -O-[CH₂]ₓ-C_{f}H_{(2f+1-g}), F_{g}, Benzyloxy, im Phenylring gegebenenfalls einfach substituiert mit Fluor, Chlor oder (C₁-C₅)-Alkoxy einschließlich der physiologisch wirksamen Salze.

In höchstem Maß bevorzugt sind Verbindungen der Formel I, in der
Q O,
X O,
Y C_{R}3,
m O,
A eine -CH₂-Gruppe,
B CO₂H,
R¹ Wasserstoff, (C₁-C₁₀)-Alkoxy, (C₅-C₆)-Cycloalkyloxy, (C₅-C₆)-Cycloalkyl-(C₁-C₂)-alkoxy, -O-[CH₂]ₓ-C_{f}H_{(2f+1-g)}F_{g}, (C₁-C₄)-Alkoxy-(C₁-C₄)-alkoxy, substituiertes Phenoxy, substituiertes Benzyloxy bedeutet, wobei der Phenylrest mit einem Substituenten aus der Reihe Fluor, Chlor, Cyano, Trifluormethyl, (C₁-C₄)-Alkyl oder (C₁-C₄)-Alkoxy substituiert ist und
R² und R³ Wasserstoff bedeuten, einschließlich der physiologisch wirksamen Salze.

In höchstem Maße bevorzugt ist weiterhin die Verbindung der Formel I, in der
Q S,
X O,
Y C_{R}3,
m O,
A eine -CH₂-Gruppe bedeutet,
B -CO₂H,
R1 Wasserstoff und
R² und R³ Wasserstoff bedeuten.

In höchstem Maße bevorzugt ist weiterhin die Verbindung der Formel I, in der
Q O,
X O,
Y C_{R}3,
m 0,
A eine -CH₂-Gruppe bedeutet,
B -C0₂H,
R¹ Wasserstoff und
R² und R³ zusammen mit dem sie tragenden Pyridin einen Chinolin-Ring bilden

Die Erfindung umfaßt weiterhin Prodrugs zu den Verbindungen der Formel (I), die eine Hemmung der Kollagenbiosynthese in vivo durch Freisetzung von Verbindungen der Formel I oder deren Salzen bewirken

Schließlich umfaßt die Erfindung auch Prodrugs, die in vivo durch Freisetzung von Verbindungen der Formel I oder deren Salzen eine inhibitorische Wirkung auf die Prolyl-4-hydroxylase bewirken.

Prodrug-Gruppierungen sind chemische Gruppen, die in vivo
zur Carboxylatgruppe der Verbindungen der Formel umgewandelt werden und/oder
vom Amid-N-Atom abgespalten werden können und/oder
zu einem Pyridinring umgewandelt werden können.

Die in Betracht kommenden Prodrug-Gruppen sind dem Fachmann bekannt.

Insbesondere sind folgende Prodrug-Gruppierungen genannt:
für die Carboxylatgruppe Ester-, Amid-, Hydroxymethyl- und Aldehydgruppen und deren Abkömmlinge, für das Pyridin-N-Atom N-Oxide und N-Alkylderivate und für den Pyridinring 1,4-Dihydro- und Tetrahydropyridin-Derivate.

Die Erfindung betrifft die Verwendung von Verbindungen der allgemeinen Formel I sowie die physiologisch verträglichen Salze zur Inhibierung der Kollagenbiosynthese.

Die Erfindung betrifft die Verwendung von Verbindungen der allgemeinen Formel I sowie die physiologisch verträglichen Salze zur Hemmung der Prolyl-4-hydroxylase.

Weiterhin betrifft die Erfindung die Verwendung von Verbindungen der allgemeinen Formel I sowie die physiologisch verträglichen Salze zur Herstellung eines Arzneimittels gegen fibrotische Erkrankungen.

Weiterhin betrifft die Erfindung die Verwendung von Verbindungen der allgemeinen Formel I sowie die physiologisch verträglichen Salze zur Herstellung eines Arzneimittels gegen fibrotische Erkrankungen der Leber, der Lunge und der Haut.

Schließlich betrifft die Erfindung die Verbindungen der allgemeinen Formel I zur Verwendung als Arzneimittel.

Insbesondere betrifft die Erfindung die Verbindungen der Formel I zur Anwendung als Fibrosuppressiva.

Weiterhin betrifft die Erfindung ein Verfahren zur Herstellung von Verbindungen der allgemeinen Formel I.

Die Herstellung der Verbindungen der Formeln I und l', in denen
x=O
A - B = -(CH₂)₁₋₄-CO₂H und
m = 0 und 1 bedeuten, erfolgt, indem
   i1.) Pyridin-2-carbonsäuren der Formel II (R¹¹ = H) mit den Aminoestern der Formel III zu den Amidestern der Formel IV umgesetzt werden, oder
   i2.) Pyridin-2-carbonsäureester der Formel II (R¹¹ = niedrig Alkyl) unter den Bedingungen der Aminolyse zu den Verbindungen der Formel IV umgesetzt werden;
   ii) die Verbindungen der Formeln I und V aus ihren Estern der Formel IV freigesetzt werden; und
   iii) die Verbindungen der Formeln IV, V oder I mit einem Oxidationsmittel zu den Pyridin-N-oxiden der Formeln l' und VI oxidiert werden und die Verbindungen IV gegebenenfalls anschließend zu den Pyridin-N-oxiden der Formel I' verseift werden.

Die Umsetzungen i1); i2) und ii) können mit Verbindungen, in denen R¹¹ = H bedeutet oder mit Verbindungen, in denen R¹¹ eine O-Schutzgruppe bedeutet, durchgeführt werden.

Geeignete Schutzgruppen, wie sie dem Fachmann geläufig sind, sind beispielsweise Methyl, Ethyl, MEM, MOM, Benzyl, 4-MeO-benzyl oder 3,4-Dimethoxybenzyl.

Weitere Schutzgruppen und die Bedingungen ihrer Abspaltung (Überführung von Verbindungen der Formel V in Verbindungen der Formel I sind von Theodoro W. Greene, Peter G.M. Wuts, in Protective Groups in Organic Synthesis, Second Edition 1991, John Wiley, Kapitel 2 und 3 beschrieben. 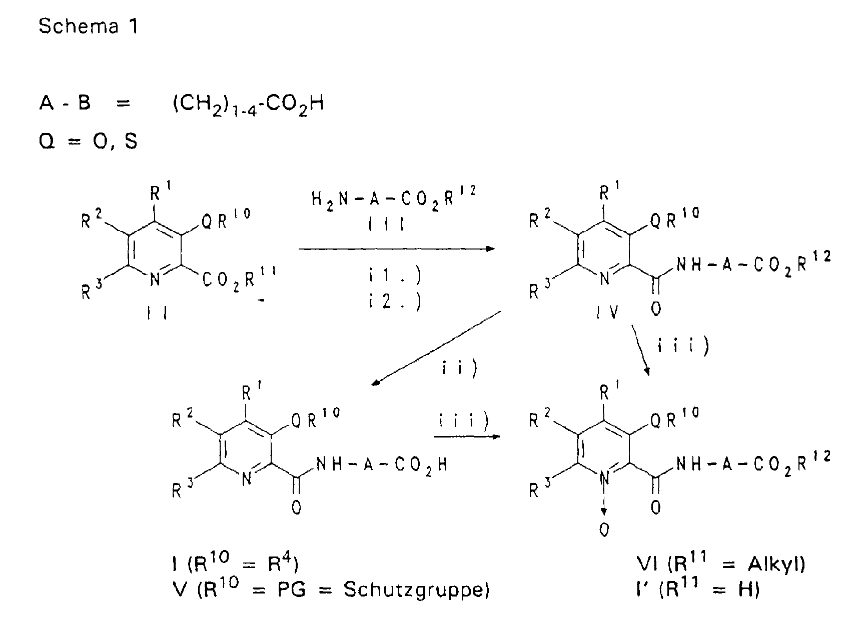
R¹⁰ ₌ R⁴ oder PG (Protecting Group)
R¹¹ = H, (C₁-C₈)-Alkyl, Benzyl
R¹²= = H, (C₁-C₈)-Alkyl, Benzyl

Geeignete Verfahren zur Amidbildung (Umsetzung ii) sind die Methoden der Carboxylaktivierung und die aus der Peptidchemie bekannten Kondensationsreaktionen.

An Reagenzien zur Carbonsäureaktivierung können die dem Fachmann bekannten Substanzen, wie Thionylchlorid, Oxalylchlorid, Pivaloylchlorid, Chlorameisensäureester-Derivate oder N,N'-Carbonyldimidazol Verwendung finden. Die aktivierten Derivate der Verbindungen der Formel II werden nach Herstellung in situ mit den Amidderivaten der Formel III umgesetzt.

Ein geeignetes Kondensationsmittel ist beispielsweise die Kombination von N,N'-Dicyclohexylcarbodiimid, 1-Hydroxy-1 H-benzotriazol und N-Ethylmorpholin.

Geeignete Lösungsmittel sind Dichlormethan, Tetrachlormethan, Butylacetat, Ethylacetat, Toluol, Tetrahydrofuran, Dimethoxyethan, 1,4-Dioxan, Acetonitril, N,N-Dimethylformamid, N,N-Dimethylacetamid, Dimethylsulfoxid, Nitromethan und/oder Pyridin.

3-Hydroxypyridin-2-carbonsäure kann käuflich erworben werden. 3-Mercaptopyridin-2-carbonsäure ist aus Roczniki Chemji 1932, 493 bekannt.

Zur Herstellung von in 4-Position (R¹) substituierten Derivaten (Q = 0) können die aus EP-A-0 304 732, EP-A-0 321 385 und EP-A-0 208 452 bekannten 2-Hydroxymethylpyridine der Formel Vlla als Zwischenprodukte Verwendung finden.

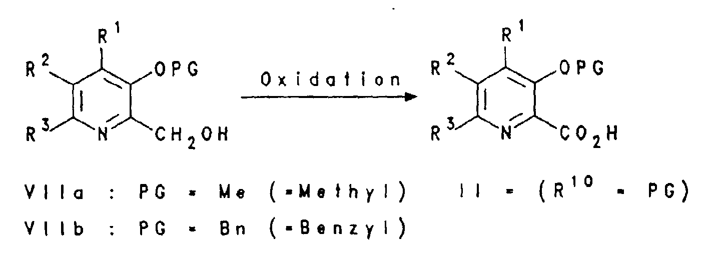

Wie dort beschrieben, werden in analoger Weise auch die 3-O-Benzylderivate der Formel Vllb erhalten.

Die Verbindungen der Formeln Vlla und Vllb wurden mit einem Oxidationsmittel, vorzugsweise mit KMnO₄ in wäßrigem alkalischen Milieu, zu den Pyridin-2-carbonsäurederivaten der Formel II (R¹⁰ = PG) umgesetzt.

Die Verbindungen der Formel I sind Inhibitoren der Prolyl-4-hydroxylase. Die Hemmung dieses Enzyms wurde, wie von Kaule und Günzler in Annal. Biochem. 184, 291 bis 297 (1990) beschrieben, bestimmt.

Die erfindungsgemäßen Verbindungen der Formel I besitzen weiterhin wertvolle pharmakologische Eigenschaften und zeigen insbesondere antifibrotische Wirksamkeit.

Die antifibrotische Wirkung kann im Modell der Tetrachlorkohlenstoff-induzierten Leberfibrose bestimmt werden. Dazu werden Ratten mit CCI₄ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben - analysiert. Die Aktivität der Fibrogenese kann durch radioimmunologische Bestimjmung von Kollagenfragmenten und Prokollagenpeptiden im Serum bestimmt werden. Die erfindungsgemäßen Verbindungen sind in diesem Modell in Konzentration 1 bis 100 mg/kg wirksam.

Die Aktivität der Fibrogenese kann durch radioimmunologische Bestimmung des N-terminalen Propeptids des Kollagen Typ- III oder der N- bzw. C-terminalen Quervernetzungsdomäne des Kollagen-Typ-IV (7s-Kollagen bzw. Typ-IV-Kollagen NC₁) im Serum bestimmt werden.

Zu diesem Zweck wurden die Hydroxyprolin-, Prokollagen-III-Peplid-,7s-Kollagen- und Typ-IV-Kollagen-NC-Konzentrationen in der Leber von
a) unbehandelten Ratten (Kontrolle)
b) Ratten, denen Tetrachlorkohlenstoff verabreicht wurden (CCI₄-Kontrolle)
c) Ratten, denen zunächst CCI₄ und anschließend eine erfindungsgemäße Verbindung verabreicht wurde

gemessen (diese Testmethode wird beschrieben von Rouiller, C., experimental toxic injury of the liver; in The Liver; C. Rouiller, Vol. 2, 5. 335 bis 476, New York, Academic Press, 1964).

Weiterhin kann eine Wirksamkeit der erfindungsgemäßen Verbindungen in folgenden Systemen nachgewiesen werden.

Hemmung der hepatischen Prolyl-4-hydroxylase in vivo:
Dieses Modell dient zum Nachweis der akuten Hemmung der Prolyl-4-hydroxylase in vivo. Dazu werden Ratten beiderlei Geschlechts (gesund bzw. mit induzierter Leberfibrose) die Prüfusubstanz bzw. das entsprechende Vehikel appliziert (intraperitoneal, intravenös, per os) und nach Substanzgabe ¹⁴C-L-Prolin (250 µCi/kg Körpergewicht) intraperitoneal verabreicht. Danach erfolgt erneut eine intraperitoneale Applikation von ¹⁴C-L-Prolin (250 µCi/kg Körpergewicht). Schließlich werden die Tiere unter Pentobarbitalnarkose entblutet und die Leber entnommen. Die Aufreinigung des hepatischen Kollagens durch Pepsinverdau und fraktionierte Ammoniumsulfatfällung erfolgte entsprechend publizierten Protokollen (Ref. 1, 2). Das gereinigte Leberkollagen wurde hydrolysiert und der Gehalt an ¹⁴C-Hydroxyprolin und ¹⁴C-Prolin durch Aminosäureanalyse mittels lonenaustauschchromatografie bestimmt. Eine Hemmung der Prolyl-4-hydroxylase ergibt sich aus einer Absenkung des Quotienten ¹⁴C-Hydroxyprolin/[¹⁴C-Hydroxyprolin + ¹⁴C-Prolin]. Als Referenzsubstanz wird 2,2'-Dipyridyl verwendet. (Ref. 1: Chojkier, M. 1986. Hepatocyte collagen production in vivo in normal rats. J. Clin. Invest. 78: 333-339 und Ref. 2: Ogata I., et al. 1991. Minor contribution of hepatocytes to collagen production in normal and early fibrotic livers. Hepatology 14: 361-367).

Hemmung der Prolyl-4-hydroxylase in Zellkulturen:
Für die Testung von Prolyl-4-hydroxylasehemmstoffen in Zellkulturen werden folgende Zelltypen verwendet:
   Normale humane Hautfibrolasten Normal human fibrolasts, NHDF), Rattenleber-Epithelzellen (rat liver epithelial cells, Ref. 1) und primäre Fettspeicherzellen aus der Rattenleber (fat storing cells, Ref. 2). Dazu werden die Zellen in Gegenwart von Hemmstoffen kultiviert. Gleichzeitig wird das in dieser Zeit neu synthetisierte Kollagen durch 4-³H-L-Prolin und ¹⁴C-Prolin metabolisch markiert. Der Einfluß der Testsubstanzen auf den Hydroxylierungsgrad des Kollagens wird anschließend entsprechend der Methode von Chojkier et al (Ref. 3) bestimmt. Als Referenzsubstanz wird 2,2'-Dipyridyl eingesetzt. (1.: Schrode, W., Mecke, D., Gebhard, R. 1990. Induction of glutamine synthetase in periportal hepatocytes by co-cultivation with a liver epithelial cell line. Eur. J. Cell. Biol. 53: 35-41, 2. Blomhoff, R., Berg T. 1990. Isolation and cultivation of rat liver stellate cells. Methods Enzymol. 190: 59-71 und 3.: Chojkier, M. Peterkofsky, B. Bateman,, J. 1980. A new method for determining the extent of proline hydroxylation by measuring changes in the ration of [4-³H]: [¹⁴C] proline in collagenase digests. Anal. Biochem. 108: 385-393).

Die Verbindungen der Formel I können als Medikamente in Form von pharmazeutischer Präparate Verwendung finden, welche sie gegebenenfalls mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 bis 25 mg/kg/Tag, vorzugsweise 1 bis 5 mg/kg/Tag oder parenteral in Dosen von 0,01 bis 5 mg/kg/Tag, vorzugsweise 0,01 bis 2,5 mg/kg/Tag, insbesondere 0,5 bis 1,0 mg/kg/ Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Unter den im folgenden beschriebenen Beispielen werden die erfindungsgemäßen Verbindungen der Formel 1 als substituierte heterocyclische Carbonsäure-glycylamide, vorzugsweise als Pyridin-2-carbonsäure-glycylamide, bezeichnet.

Unter dieser Bezeichnungsweise werden substituierte Pyridin-2-carbonsäure-N-(carboxymethyl)amide verstanden. Die Klassifizierung als substituierte N-(Pyridyl-2-carbonyl)glycine ist eine weitere Möglichkeit.

### Beispiel 1

3-Hydroxypyridin-2-carbonsäureglycylamid

### a) 3-Benzyloxy-pyridin-2-carbonsäure

Zu 1,76 g (44 mmol) Natriumhydrid (60 % in Mineralöl) in 35 ml wasserfreiem N-N-Dimethylformamid gab man bei 25°C 2,78 g (20 mmol) 3-Hydroxypyridin-2-carbonsäure. Man rührte 1 Stunde bei 25°C, gab 5,06 ml (44 mmol) Benzylchlorid hinzu, erhitzte 2 Stunden zum Sieden, kühlte auf 25°C, engte im Vakuum ein, nahm den Rückstand in Diethylether auf, extrahierte zweimal mit Wasser, mit gesättigter wäßriger Natriumbicarbonat-Lösung und mit gesättigter Natriumchlorid-Lösung. Nach Trocknen und Einengen im Vakuum der organischen Phase wurde das braune Rohprodukt mit n-Heptan/Ethylacetat(1: 1) an Kieselgel chromatographiert. Die so erhaltenen 2,46 g (17 mmol) des Benzylesters wurden in Tetrahydrofuran/Methanol (2:1) mit 17 ml (17 mmol) 1 N Natronlauge verseift. Nach 48 Stunden bei 25°C gab man Zitronensäure zu, engte im Vakuum ein und chromatographierte mit Ethylacetat/Methanol (1:1) an Kieselgel. Man erhielt 1,38 g Produkt in Form blaßgelber Kristalle, Fp. 64-66°C.

### b) 2-Benzyloxy-pyridin-2-carbonsäure-(glycylmethylester)amid

1,38 g (6 mmol) der vorstehenden Carbonsäure wurden in 30 ml wasserfreiem Tetrahydrofuran bei 0°C 30 Minuten mit 1,7 ml (12,1 mmol) Triethylamin gerührt. Dann gab man 0,81 ml (66 mmol) Pivaloylchlorid zu, rührte 3 Stunden bei 0°C, gab 0,92 g (6,6 mmol) Glycinmethylester-Hydrochlorid und 0,85 ml (6 mmol) Triethylamin zu, rührte 1 Stunde bei 0°C, erwärmte auf 20°C, engte im Vakuum ein, nahm den Rückstand in 100 ml Ethylacetat auf, extrahierte mit gesättigter wäßriger Natriumbicarbonat-Lösung und gesättigter NaCI-Lösung, engte nach dem Trocknen im Vakuum ein und reinigte den Rückstand durch Flash-Chromatographie mit Ethylacetat/Methanol (10: 1) an Kieselgel. Man erhielt 1,02 g blaß rosa gefärbtes Öl.

### c) 3-Hydroxypyridin-2-carbonsäure-(glycylmethylester)amid

1,02 g (3,4 mmol) des vorstehenden Benzylesters wurden in 50 ml Methanol gelöst, mit 50 mg Pd/C (10 % Pd) versetzt und in einer Schüttelente hydriert bis keine Wasseraufnahme mehr erfolgte (Ausnahme ca. 60 ml Wasserstoff). Man filtrierte über Celite, engte im Vakuum ein, reinigte den Rückstand durch Flash-Chromatographie mit EthylacetaVn-Heptan (3: 1) an Kieselgel und erhielt 580 mg farblos kristallines Produkt, Fp. 59-61°C.

d) Die Titelverbindung wurde erhalten, indem 0,45 g (2,1 mmol) des vorstehenden Methylesters in 15 ml Tetrahydrofuran/Methanol (21:1) gelöst und mit 5 ml 1 N wäßriger Natronlauge versetzt wurden. Man rührte 24 Stunden bei 20°C, engte im Vakuum ein, nahm den Rückstand in Wasser auf und säuerte mit 2N Salzsäure auf pH 3-4 an. Hierbei kristallisierte das Produkt in Form farbloser Kristalle, die nach Absaugen in der IR-Trocknungsapparatur von Wasserresten befreit wurden. Man erhielt 185 mg der Titelverbindung, Fp. 182-174°C.

### Beispiel 2

3-Hydroxypyridin-2-carbonsäure-(ß-alanyl)amid

### a) 3-Hydroxypyridin-2-carbonsäure-(ß-alanylmethylester)amid

2,78 g (20 mmol) 3-Hydroxypyridin-2-carbonsäure wurden in 80 ml wasserfreiem Tetryhydrofuran suspendiert, mit 2,72 g (20 mmol) β-Alaninmethylester-Hydrochlorid, 2,55 ml (20 mmol) N-Ethylmorpholin und 5,41 g (40 mmol) 1-Hydroxy-1 H-benzotriazol versetzt, auf 0°C abgekühlt und mit 4,33 g (21 mmol) N,N'-Dicyclohexylcarbondiimid in 20 ml Tetrafuran versetzt Dann wurden 70 Minuten bei 0°C und 60 Minuten bei 20°C gerührt, der ausfallende Feststoff abfiltriert, das Filtrat im Vakuum eingeengt, in 100 ml Dichlormethan aufgenommen, mit Wasser gewaschen, die organische Phase getrocknet, eingeengt und der Rückstand mit Ethylacetat/Methanol (5:1) an Kieselgel gereinigt, 1,24 farbloses Öl.

b) Die Titelverbindung wurde erhalten, indem 1,2 g (5,4 mmol) des vorstehenden Methylesters in 100 ml Ethanol/ Tetrahydrofuran (1:1) mit 10 ml 1N wäßriger Natronlauge 5 Stunden bei 20°C verseift wurden. Man engte im Vakuum ein, nahm in Wasser auf, extrahierte dreimal mit je 25 ml Dichlormethan. Die wäßrige Phase wurde auf pH 2 angesäuert, der entstandene Niederschlag abgesaugt und mit kaltem Wasser und mit kaltem Diethylether gewaschen. Man erhielt 0,6 g der Titelverbindung, Fp. 206°C (Zers.).

### Beispiel 3

3-Hydroxy-4-methoxypyridin-2-carbonsäure-glyclylamid

### a) 3-Benzyloxy-4-hydroxy-2-methylpyridin

200 g (1,6 mol) 3-Hydroxy-2-methyl-4-pyranon (Maltol) wurden 1 Stunde in 800 ml 2N Natronlauge (1,6 mol) gelöst und mit einer Lösung von 343 g (237 ml, 2,0 mol) Benzylbromid in 250 ml Tetrahydrofuran versetzt. Die DC-Kontrolle zeigte nach 15 Minuten ca. 25 % Umsatz. Dann wurde das Reaktionsgemisch 2 Stunden bei 60°C gerührt, 12 Stunden bei 20°C stehen lassen, die obere Phase abgetrennt, die untere mit Diethylether extrahiert und die organischen Phasen gemeinsam im Vakuum eingeengt. Zu dem erhaltenen Eindampf-Rückstand gab man 1 I konzentrierten wäßrigen Ammoniak und 500 ml 1,4-Dioxan und erwärmte auf dem Dampfbad. Nach jeweils 60 Minuten gab man sechsmal je 250 ml Ammoniaklösung nach. Nach 8 Stunden zeigte die DC-Kontrolle vollständigen Umsatz. Nach dem Abkühlen wurde die untere braune Phase abgetrennt, das Produkt durch Zugabe von Ethylacetat zur Kristallisation gebracht, abgesaugt, mit Ethylacetat gewaschen und getrocknet. Man erhielt 230 g Produkt, Fp. 165-167°C. Aus der wäßrigen Mutterlauge konnten weitere 45 g Produkt erhalten werden.

### b) 3-Benzyloxy-4-chlor-2-methylpyridin-1-oxid

21 g (0,098 mol) 3-Benzyloxy-4-hydroxy-2-methylpyridin wurden unter Rühren bei 25°C in 200 ml Phosphoroxychlorid eingetragen und 7 Stunden rückfließend erhitzt. Anschließend wurde Phosphoroxychlorid im Vakuum abdestilliert und der Rückstand portionsweise in 1 I Wasser eingetragen, von wenig Ungelöstem abgetrennt, die wäßrige Phase dreimal mit je 200 ml Dichlormethan extrahiert, die organische Phase mit Magnesiumsulfat getrocknet, eingeengt, mit Diethylether behandelt, 1 g kristallines Produkt (als Hydrochlorid) abgesaugt, Fp. 148-150°C und 10 g (43 mmol) öliges Produkt isoliert. Dieses wurde in 100 ml Dichlormethan gelöst, bei 25°C unter Rühren portionsweise 12,5 3-Chlorperbenzoesäure (50 mmol) hinzugegeben und 1 Stunde gerührt. Dann leitete man Ammoniak-Gas ein, saugte die ausgefallenen Ammoniumsalze ab, wusch diese mit Dichlormethan, leitete ein zweites Mal Ammoniak-Gas ein, filtrierte von wenig Kristallinem ab, engte im Vakuum ein und behandelte den Rückstand mit Diisopropylether. Man erhielt 8,0 g Produkt.

### c) 3-Benzyloxy-4-methoxy-2-methylpyridin-1-oxid

8 g (32 mmol) der vorstehenden Verbindung wurden unter Rühren bei 25°C portionsweise in 200 ml methanolische Natriummethylat-Lösung (aus 0,83 g (36 mmol) Na). Nachdem 2 Stunden rückfließend erhitzt wurde, zeigte die DC-Kontrolle (Ethylacetat/Methanol = 5:1) 20 % Umsatz. Man gab 5,7 ml (32 mmol) 30 %ige NaOMe-Lösung in Methanol hinzu, erhitzte 4 Stunden zum Sieden, fügte die gleiche Menge NaOMe-Lösung noch einmal hinzu und erhitzte weitere 4 Stunden zum Sieden. Nach dem Abkühlen auf 25°C wurde im Vakuum eingeengt, der Rückstand mit 100 ml Wasser versetzt, dreimal mit je 100 ml Dichlormethan extrahiert, die organische Phase getrocknet, eingeengt und der Rückstand mit Diisopropylether zur Kristallisation gebracht. Man erhielt 6,0 g Produkt, Fp. 84-86°C.

### d) 3-Benzloxy-2-hydroxymethyl-4-methoxypyridin

6,0 g (25,5 mmol) des vorstehenden N-Oxids wurden in 20 ml Eisessig gelöst und bei 80°C unter Rühren tropfenweise mit 30 ml Acetanhydrid versetzt, sodann 1 Stunde bei 90°C erhitzt. DC-Kontrolle (Ethylacetat/Methanol 5:1) zeigte 20 % Umsatz. Nach weiteren 30 Minuten bei 120°C war die Reaktion vollständig. Man kühlte auf 80°C, versetzte mit 15 ml Methanol, erhitzte 15 Minuten zum Sieden, klärte über Aktivkohle, engte im Vakuum ein, fügte den Rückstand, in wenig Methanol gelöst, zu 200 ml 1,5N methanolischer NaOH. Nach 1 Stunde wurde im Vakuum eingeengt, der Rückstand in 200 ml Wasser aufgenommen, dreimal mit je 150 ml Ethylacetat ausgeschüttelt, die organische Phase über MgS0₄ getrocknet und eingeengt. Man erhielt 4,4 g öliges Rohprodukt, das weiter umgesetzt wurde.

### e) 3-Benzyloxy-4-methoxypyridin-2-carbonsäure

4,4 g (18 mmol) der vorstehenden 2-Hydroxymethylverbindung und 1,6 g Kaliumhydroxid wurde in 100 ml Wasser gelöst und unter Rühren in 2 Portionen mit 2,6 g (16,3 mmol) Kaliumpermanganat versetzt. Nach 15 Minuten wurden 1,95 g Kaliumpermanganat hinzugegeben und 30 Minuten bei dieser Temperatur gerührt. Ausgefallenes Mn0₂ wurde von der heißen Lösung abgesaugt, zweimal mit heißem Wasser nachgewaschen, das Filtrat im Vakuum auf 50 ml eingeengt und unter Eiskühlung mit konzentriertem HCI auf pH 1 gebracht. Die kristalline Fällung wurde abgesaugt und getrocknet; 4,6 g Produkt, Fp. 224-225°C.

### f) 3-Benzyloxy-4-methoxypyridin-2-carbonsäure-(glyclylethylester)amid

2,6 g (10 mmol) der vorstehenden Pyridin-2-carbonsäure wurden in 250 ml wasserfreiem Dichlormethan und 80 ml wasserfreiem Tetrahydrofuan gelöst und unter Rühren mit 1,4 g (10 mmol) Glycinethylester-hydrochlorid, 2,8 ml (22 mmol) N-Ethyl-morpholin, 1,5 g (11 mmol) 1-Hydroxy-1H-benzotriazol, 2,3 g (11 mmol) N-N'-Dicyclohexylcarbodiimid versetzt und 20 Minuten bei 20°C gerührt. Anschließend wurde von Ungelösten abfiltriert, im Vakuum eingeengt, der Rückstand in 200 ml Dichlormethan gelöst mit 200 ml gesättigter wäßriger Natriumbicarbonat-Lösung gerührt, die organische Phase abgetrennt, getrocknet, eingeengt und der Rückstand mit Ethylacetat an Kieselgel chromatographiert. Man erhielt 2,0 g öliges Produkt, das unter g) sofort weiter umgesetzt wurde.

### g) 3-Hydroxy-4-methoxypyridin-2-carbonsäure(glycylethylester)amid

2,0 g (5,8 mmol) des vorstehenden Benzylethers wurden in 100 ml Tetrahydrofuran/Methanol (1:1) gelöst und mit Pd/C (10 %) in der Schüttelente hydriert. Nachdem 130 ml Wasserstoff aufgenommen worden waren, wurde der Katalysator abgesaugt, das Filtrat im Vakuum eingeengt und der Rückstand mit Diisopropylether zur Kristallisation gebracht. Man erhielt 1,2 g Produkt, Fp. 97-99°C.

h) Die Titelverbindung wurde erhalten, indem 0,5 g (1,97 mmol) des vorstehenden Ethylesters unter Rühren in 100 ml 1,5 N methanolische NaOH eingetragen wurden und 30 Minuten bei 20°C gerührt wurde. Dann wurde im Vakuum eingeengt, der Rückstand in Wasser gelöst und mit konzentriertem HCI auf pH 1 gebracht. Da keine Kristallisation erfolgte, engte man erneut ein, behandelte den Rückstand zweimal mit wasserfreiem Ethanol einmal mit Diethylether, saugte jeweils vom Rückstand ab, destillierte die organischen Lösungsmittel im Vakuum ab und brachte den Rückstand mit Diethylether zur Kristallisation. Man erhielt 160 mg der Titelverbindung, Fp. 270-271 °C.

### Beispiel 4

3-Hydroxy-4-methoxypyridin-2-carbonsäure-L-alanylamid

### Beispiel 5

4-Hexyloxy-3-hydroxypyridin-2-carbonsäuro-glycylamid

### Beispiel 6

4-(3-Ethyloxypropyloxy)-3-hydroxy-pyridin-2-carbonsäure-glycylamid

### Beispiel 7

3-Hydroxy-4-(2,2,2-trifluorethyloxy)pyridin-2-carbonsäure-glycylamid

### Beispiel 8

4-Ethyloxy-3-hydroxypyridin-2-carbonsäure-glycylamid

### Beispiel 9

4-Butyloxy-3-hydroxypyridin-2-carbonsäuro-glycylamid

### Beispiel 10

3-Hydroxy-4-propyloxypyridin-2-carbonsäure-glycylamid

### Beispiel 11

3-Hydroxy-4-(2-propyloxy)pyridin-2-carbonsäure-glycylamid

### Beispiel 12

3-Hydroxy-4-(2-methylpropyloxy)pyridin-2-carbonsäure-glycylamid

### Beispiel 13

3-Hydroxy-4-pentyloxypyridin-2-carbonsäure-glycylamid

### Beispiel 14

3-Hydroxy-4-(3-methylbutyloxy)pyridin-2-carbonsäure-glycylamid

### Beispiel 15

4-(2-Ethylbutyloxy)-3-hydroxypyridin-2-carbonsäure-glycylamid

### Beispiel 16

4-(2-Cyclohexylethyloxy)-3-hydroxypyridin-2-carbonsäuro-glycylamid

### Beispiel 17

4-(Cyclohexylmethyloxy)-3-hydroxypyridin-2-carbonsäure-glycylamid

### Beispiel 18

4-Cyclohexyloxy-3-hydroxypyridin-2-carbonsäure-glycylamid

### Beispiel 19

3-Hydroxy-4-(3-methoxypropyloxy)pyridin-2-carbonsäure-glycylamid

### Beispiel 20

3-Hydroxy-4-(2-phenoxyethyloxy)pyridin-2-carbonsäure-glycylamid

### Beispiel 21

4-Benzyloxy-3-hydroxypyridin-2-carbonsäure-glycylamid

### Beispiel 22

4-(4-Chlorbenzyloxy)-3-hydroxypyridin-2-carbonsäure-glycylamid

### Beispiel 23

4-(4-Fluorbenzyloxy)-3-hydroxypyridin-2-carbonsäure-glycylamid

### Beispiel 24

3-Hydroxy-4-(4-trifluormethylbenzyloxy)pyridin-2-carbonsäure-glycylamid

### Beispiel 25

3-Hydroxy-4-(3-trifluormethylbenzyloxy)pyridin-2-carbonsäure-glycylamid

### Beispiel 26

3-Hydroxy-4-(4-trifluormethoxybenzyloxy)pyridin-2-carbonsäure-glycylamid

### Beispiel 27

4-(3,5-Bis(trifluormethyl)benzyloxy)-3-hydroxypyridin-2-carbonsäure-glycylamid

### Beispiel 28

4-(3,5-Dichlorbenzyloxy)-3-hydroxypyridin-2-carbonsäure-glycylamid

### Beispiel 29

3-Hydroxy-4-(2,2,3,3,3-pentafluorpropyloxy)pyridin-2-carbonsäuro-glycylamid

### Beispiel 30

4-(2,2,3,3,4,4,4-Heptafluorbutyloxy)-3-hydroxypyridin-2-carbonsäure-glycylamid

### Beispiel 31

3-Hydroxy-4-(2,2,3,3-tetrafluorpropyloxy)pyridin-2-carbonsäure-glycylamid

### Beispiel 32

4-Ethoxy-3-hydroxypyridin-2-carbonsäure-L-alanylamid

### Beispiel 33

4-Butyloxy-3-hydroxypyridin-2-carbonsäure-L-alanylamid

### Beispiel 34

3-Hydroxy-4-propyloxypyridin-2-carbonsäure-L-alanylamid

### Beispiel 35

3-Hydroxy-4-(2-propyloxy)pyridin-2-carbonsäure-L-alanylamid

### Beispiel 36

3-Hydroxy-4-(2-methylpropyloxy)pyridin-2-carbonsäure-L-alanylamid

### Beispiel 37

3-Hydroxy-4-pentyloxypyridin-2-carbonsäure-L-alanylamid

### Beispiel 38

3-Hydroxy-4-(3-methylbutyloxy)pyridin-2-carbonsäure-L-alanylamid

### Beispiel 39

4-(2-Ethylbutyloxy)-3-hydroxypyridin-2-carbonsäure-L-alanylamid

### Beispiel 40

4-(2-Cyclohexylethyloxy)-3-hydroxy-pyridin-2-carbonsäure-L-alanylamid

### Beispiel 41

4-(Cyclohexylmethyloxy)-3-hydroxypyridin-2-carbonsäure-L-alanylamid

### Beispiel 42

4-Cyclohexyloxy-3-hydroxypyridin-2-carbonsäure-L-alanylamid

### Beispiel 43

3-Hydroxy-4-(3-methoxypropyloxy)pyridin-2-carbonsäure-L-alanylamid

### Beispiel 44

3-Hydroxy-4-(2-phenoxyethyloxy)pyridin-2-carbonsäure-L-alanylamid

### Beispiel 45

4-Benzyloxy-3-hydroxypyridin-2-carbonsäure-L-alanylamid

### Beispiel 46

4-(4-Chlorbenzyloxy)-3-hydroxypyridin-2-carbonsäure-L-alanylamid

### Beispiel 47

4-(4-Fluorbenzyloxy)-3-hydroxypyridin-2-carbonsäu re-L-alanylamid

### Beispiel 48

3-Hydroxy-4-(4-trifluormethylbenzyloxy)pyridin-2-carbonsäure-L-alanylamid

### Beispiel 49

3-Hydroxy-4-(3-trifluormethylbenzyloxy)pydridin-2-carbonsäure-L-alanylamid

### Beispiel 50

3-Hydroxy-4-(4-trifluormethoxybenzyloxy)pyridin-2-carbonsäure-L-alanylamid

### Beispiel 51

4-(3,5-Bis(trifluormethyl)benzyloxy)-3-hydroxypyridin-2-carbonsäure-L-alanylamid

### Beispiel 52

4-(3,5-Dichlorbenzyloxy)-3-hydroxypyridin-2-carbonsäure-L-alanylamid

### Beispiel 53

3-Hydroxy-4-(2,2,2-trifluorethyloxy)pyridin-2-carbonsäure-L-alanylamid

### Beispiel 54

3-Hydroxy-4-(2,2,3,3,3-pentafluorpropyloxy)pyridin-2-carbonsäuro-L-alanylamid

### Beispiel 55

4-(2,2,3,3,4,4,4-Heptafluorbutyloxy)-3-hydroxypyridin-2-carbonsäure-L-alanylamid

### Beispiel 56

3-Hydroxy-4-(2,2,3,3-tetrafluorpropyloxy)pyridin-2-carbonsäure-L-alanylamid

### Beispiel 57

4-Ethyloxy-3-hydroxypyridin-2-carbonsäure-L-phenylalanylamid

### Beispiel 58

4-Butyloxy-3-hydroxypyridin-2-carbonsäure-L-phenylalanylamid

### Beispiel 59

3-Hydroxy-4-propyloxypyridin-2-carbonsäure-L-phenylalanylamid

### Beispiel 60

3-Hydroxy-4-(2-propyloxy)pyridin-2-carbonsäure-L-phenylalanylamid

### Beispiel 61

3-Hydroxy-4-(2-methylpropyloxy)pyridin-2-carbonsäure-L-phenylalanylamid

### Beispiel 62

3-Hydroxy-4-pentyloxypyridin-2-carbonsäure-L-phenylalanylamid

### Beispiel 63

3-Hydroxy-4-(3-methylbutyloxy)pyridin-2-carbonsäure-L-leucylamid

### Beispiel 64

4-(2-Ethylbutyloxy)-3-hydroxypyridin-2-carbonsäure-L-leucylamid

### Beispiel 65

4-(2-Cyclohexylethyloxy)-3-hydroxypyridin-2-carbonsäure-L-leucylamid

### Beispiel 66

4-(Cyclohexylmethyloxy)-3-hydroxypyridin-2-carbonsäure-L-leucylamid

### Beispiel 67

4-Cyclohexyloxy-3-hydroxypyridin-2-carbonsäuro-L-leucylamid

### Beispiel 68

3-Hydroxy-4-(3-methoxypropyloxy)pyridin-2-carbonsäure-L-leucylamid

### Beispiel 69

3-Hydroxy-4-(2-phenoxyethyloxy)pyridin-2-carbonsäure-L-leucylamid

### Beispiel 70

4-Ethyloxy-3-hydroxypyridin-2-carbonsäure-D-alanylamid

### Beispiel 71

4-Butyloxy-3-hydroxypyridin-2-carbonsäure-D-alanylamid

### Beispiel 72

3-Hydroxy-4-propyloxypyridin-2-carbonsäure-D-alanylamid

### Beispiel 73

3-Hydroxy-4-(2-propyloxy)pyridin-2-carbonsäure-D-alanylamid

### Beispiel 74

3-Hydroxy-4-(2-methylpropyloxy)pyridin-2-carbonsäure-D-phenylalanylamid

### Beispiel 75

3-Hydroxy-4-pentyloxypyridin-2-carbonsäure-D-phenylalanylamid

### Beispiel 76

3-Hydroxy-4-(3-methylbutyloxy)pyridin-2-carbonsäure-L-valylamid

### Beispiel 77

4-(2-Ethylbutyloxy)-3-hydroxypyridin-2-carbonsäure-L-valylamid

### Beispiel 78

4-(2-Cyclohexylethyloxy)-3-hydroxypyridin-2-carbonsäure-L-valylamid

### Beispiel 79

4-(Cyclohexylmethyloxy)-3-hydroxypyridin-2-carbonsäure-L-serylamid

### Beispiel 80

4-Cyclohexylmethyloxy-3-hydroxypyridin-2-carbonsäure-L-norleucylamid

### Beispiel 81

3-Hydroxy-4-(3-methoxypropyloxy)pyridin-2-carbonsäure-D-alanylamid

### Beispiel 82

3-Hydroxy-4-(2-phenoxyethyloxy)pyridin-2-carbonsäure-D-alanylamid

### Beispiel 83

6-Ethyloxy-3-hydroxypyndin-2-carbonsäure-glycylamid

### Beispiel 84

6-Butyloxy-3-hydroxypyridin-2-carbonsäure-glycylamid

### Beispiel 85

3-Hydroxy-6-propyloxypyridin-2-carbonsäure-glycylamid

### Beispiel 86

3-Hydroxy-6-(2-propyloxy)pyridin-2-carbonsäure-glycylamid

### Beispiel 87

3-Hydroxy-6-(2-methylpropyloxy)pyridin-2-carbonsäure-glycylamid

### Beispiel 88

3-Hydroxy-6-pentyloxypyridin-2-carbonsäure-glycylamid

### Beispiel 89

3-Hydroxy-6-(3-methylbutyloxy)pyridin-2-carbonsäure-glycylamid

### Beispiel 90

6-(2-Ethylbutyloxy)-3-hydroxypyridin-2-carbonsäure-glycylamid

### Beispiel 91

6-(2-Cyclohexylethyloxy)-3-hydroxypyridin-2-carbonsäure-L-alanylamid

### Beispiel 92

6-(Cyclohexylmethyloxy)-3-hydroxypyridin-2-carbonsäure-glycylamid

### Beispiel 93

6-Cyclohexyloxy-3-hydroxypyridin-2-carbonsäure-glycylamid

### Beispiel 94

3-Hydroxy-6-(3-methoxypropyloxy)pyridin-2-carbonsäure-glycylamid

### Beispiel 95

3-Hydroxy-6-(2-phenoxyethyloxy)pyridin-2-carbonsäure-glycylamid

### Beispiel 96

6-Benzyloxy-3-hydroxypyridin-2-carbonsäure-glycylamid

### Beispiel 97

6-(4-Chlorobenzyloxy)-3-hydroxypyridin-2-carbonsäure-glycylamid

### Beispiel 98

6-(4-Fluorbenzyloxy)-3-hydroxypyridin-2-carbonsäure-glycylamid

### Beispiel 99

3-Hydroxy-6-(2,2,2-trifluorethyloxy)pyridin-2-carbonsäure-glycylamid

### Beispiel 100

6-(2,2,3,3,4,4,4-Heptafluorbutyloxy)-3-hydroxypyridin-2-carbonsäuro-glycylamid

### Beispiel 101

3-Hydroxy-4-morpholinylpyridin-2-carbonsäure-glycylamid

### Beispiel 102

3-Hydroxy-4-piperidylpyridin-2-carbonsäure-glycylamid

### Beispiel 103

3-Hydroxy-4-pyrollidinylpyridin-2-carbonsäure-glycylamid

### Beispiel 104

4-Dimethylamino-3-hydroxypyridin-2-carbonsäure-glycylamid

### Beispiel 105

5-Chlor-3-hydroxypyridin-2-carbonsäure-glycylamid

### Beispiel 106

5-Cyano-3-hydroxypyridin-2-carbonsäure-glycylamid

### Beispiel 107

3-Hydroxy-6-piperidylpyridin-2-carbonsäure-glycylamid

### Beispiel 108

3-Hydroxy-6-morpholinylpyridin-2-carbonsäure-glycylamid

### Beispiel 109

5-Carboxy-3-hydroxypyridin-2-carbonsäure-N-(carboxymethyl)amid

### Beispiel 110

5-((1-Hexyloxy)carbonyl)-3-hydroxypyridin-2-carbonsäure-N-(carboxymethyl)amid

### Beispiel 111

N-(3-Hydroxy-1 -oxypyridin-2-carbonyl)glycin

### a) N-(3-Hydroxypyridin-2-carbonyl)glycinmethylester

3-Hydroxypyridin-2-carbonsäure (6,96 g, 50 mmol), 6,28 g Glycinmethylester Hydrochlorid, 6,4 ml N-Ethyl- morpholin und 13,53 g 1-Hyroxy-1H-benzotriazol wurden in 300 ml wasserfreiem Dichlormethan suspendiert und bei 0°C mit einer Lösung von 10,83 g DCC (= N,N'-Dicyclohexylcarbodiimid) in 50 ml Dichlormethan versetzt. Nach 12 Stunden wurde filtriert und das Filtrat mit Wasser und gesättigter wäßriger NaHC0₃-Lösung gewaschen. Trocknen und Einengen ergab 7,33 g (70 %) N-(3-Hydroxypyridin-2-carbonyl)glycinmethylester, Fp. 59-61 °C (Ethylacetat/Heptan), vgl. auch Beispiel 1c).

### b) N-(3-Hydroxy-1-oxypyridin-2-carbonyl)glycinmethylester

N-(3-Hydroxy-2-carbonyl)glycinmethylester (7,33 g, 34,9 mmol), wurden in 200 ml Dichlormethan gelöst und mit 12,42 g (72 mmol) m-Chlorperbenzoesäure versetzt. Nach 24 Stunden filtrierte man ab. Das Filtrat wurde mit Wasser und NaHC0₃-Lösung gewaschen und eingeengt. Chromatographie des Rückstandes mit E thylacetat/ Heptan (3:1) lieferte 0,8 g (10 %) N-(3-Hydroxy-1-oxypyridin-2-carbonyl)glycinmethylester in Form von farblosen Kristallen; Fp. 126-128°C.

c) Die Titelverbindung wurde erhalten, indem N-(3-Hydroxy-1-oxypyridin-2-carbonyl)glycinmethylester (0,2 g, 0,9 mmol) in 5 ml Tetrahydrofuran und 10 ml Ethanol gelöst und mit 1,5 ml 1 N wäßriger Natronlauge versetzt wurden. Nach 4 Stunden engte man im Vakuum ein, nahm den Rückstand in 10 Wasser auf und extrahierte die organische Phase mit Dichlormethan. Die wäßrige Phase wurde mit Salzsäure vorsichtig auf pH 3 angesäuert und gekühlt. Hierbei fiel die Titelverbindung in Form von farblosen Kristallen an (0,078 g, 42 %). Fp. 186°C (aus wäßriger Salzsäure).

### Beispiel 112

3-Mercaptopyridin-2-carbonsäure-N-(carboxymethyl)amid

### a) 3-Mercaptopyridin-2-carbonsäure

wurde hergestellt nach E. Sucharda, Cz. Troszkiewiczöwna, Roczniki Chemiji 1932, 493.

### b) 3-Mercaptopyridin-2-carbonsäure-N-((methoxycarbonyl)methyl)amid

1,69 g 3-Mercaptopyridin-2-carbonsäure wurden in 20 ml DMF gelöst und unter Rühren bei Raumtemperatur nacheinander 7,5 ml N-Ethylmorpholin, 1,7 g 1-Hydroxy-1H-benzotriazol, 5 g N-Cyclohexyl-N'-(2-morpholinoe- thyl)-carbodiimid-methyl-p-toluolsulfonat und 4,3 g Glycinmethylester-Hydrochlorid zugegeben. Nach 12-stündigem Rühren wurde das Gemisch zwischen gesätt. wäßriger Ammoniumchloridlösung und Dichlormethan verteilt, die organische Phase getrocknet, eingedampft und der Rückstand durch Chromatographie an Kieselgel (Eluens: Ethylacetat/Heptan) gereinigt; Ausbeute 0,65 g farblose Kristalle vom Fp. 178-179°C (Ethylacetat/Heptan).

c) 226 mg der vorstehenden Verbindung wurden in 20 ml 1,4-Dioxan gelöst und 1,1 ml 1M wäßrige LiOH-Lösung zugegeben. Nach mehrstündigem Rühren bei Raumtemperatur wurde mit Essigsäure angesäuert, mit Dichlormethan mehrfach extrahiert, die organischen Phasen getrocknet und eingedampft. Der Rückstand wurde durch Chromatographie an Kieselgel (Eluens: Ethylacetat/Essigsäure) gereinigt. Ausbeute 25 mg farblose Kristalle, Fp. 252-254°C (aus 2-Propanol).

### Beispiel 113

### 3-Hydroxychinolin-2-carbonsäure-N-(carboxymethyl)amid

a) 3-(2-Nitrobenzoyl)acetylaceton wurde aus Acetylaceton und 2-Nitrobenzoylchorid erhalten, Fp. 69°C; vgl. J. Prakt. Chem. 1987, 329, S. 1063, 29 % Ausbeute.
b) 2-Acetyl-3-hydroxychinolin wurde aus dem Produkt a) unter basischen Bedingungen (KOH/Wasser, Smiles-Umlagerung) erhalten, Fp. 105°C; vgl. J. Chem. Soc. Chem. Comm. 1975, 782; 53 % Ausbeute.
c) 2-Acetyl-3-benzyloxychinolin wurde aus dem Produkt b) mit Benzylbromid (Pottasche/Aceton) erhalten, 52 % Ausbeute,
   ¹H-NMR (CDCI₃): 8 = 2,89 (s, 3 H), 5,25 (s, 2 H), 7,38 (m, 3 H), 7,58 (m, 5 H), 7,70 (m, 1 H), 8,08 (m, 1 H).
d) 3-Benzyloxychinolin-2-carbonsäure wurde aus dem Produkt c) mit Kaliumhypochlorit (Dioxan/Wasser) erhalten, öliges Rohprodukt, 47 % Ausbeute,
   ¹ H-NMR (CDCI₃): δ = 5,40 (s, 2 H), 7,40 (m, 3 H), 7,63 (m, 4 H), 7,75 (m, 2 H), 8,07 (m, 1 H).
e) 3-Benzyloxychinolin-2-carbonsäure-N-((benzyloxycarbonyl)methyl)amid wurde aus dem Produkt d) mit Triethylamin/Chlorameisensäureethylester (gemischte Anhydrid-Methode) und Glycinbenzylester-Tosylat erhalten, öliges Rohprodukt, 64 % Ausbeute,
   ¹ H-NMR (CDCI₃): δ= 4,40 (d, 2 H), 5,25 (s, 2 H), 5,35 (s, 2 H), 7,10 bis 7,75 (m, 14 H), 8,10 (m, 1 H), 7,28 (t, 1 H).
f) Die Titelverbindung wurde erhalten, indem das Produkt e) in Methanol mit Pd/C (10 %) in der Schüttelente hydriert wurde, Fp. 191 °C (aus wäßriger Salzsäure), 40 % Ausbeute.

Die folgenden Beispiele wurden analog Beispiel 113 hergestellt:

### Beispiel 114

N-(3-Hydroxy-6-methoxychinolin-2-carbonyl)glycin

### Beispiel 115

N-(6-Ethyloxy-3-hydroxychinolin-2-carbonyl)glycin

### Beispiel 116

N-(6-(1-Butyloxy)-3-hydroxychinolin-2-carbonyl)glycin

### Beispiel 117

N-(6-(1-Hexyloxy)-3-hydroxychinolin-2-carbonyl)glycin

### Beispiel 118

N-(3-Hydroxy-6-(1-octyloxy)chinolin-2-carbonyl)glycin

### Beispiel 119

N-(6-(1-Decyloxy)-3-hydroxychinolin-2-carbonyl)glycin

### Beispiel 120

N-(3-Hydroxy-6-((2,2,2-trifluorethyl)oxy)chinolin-2-carbonyl)glycin

### Beispiel 121

N-(3-Hydroxy-6-((2,2,3,3,3-pentafluorpropyl)oxy)chinolin-2-carbonyl)glycin

### Beispiel 122

N-(6-((2,2,3,3,4,4,4-Heptafluorbutyl)oxy)-3-hydroxychinolin-2-carbonyl)glycin

### Beispiel 123

N-(6-Chlor-3-hydroxychinolin-2-carbonyl)glycin

### Beispiel 124

N-(6-Brom-3-hydroxychinolin-2-carbonyl)glycin

### Beispiel 125

N-(3-Hydroxy-6-(4-phenylsulfonyl))chinolin-2-carbonyl)glycin

### Beispiel 126

N-(6-((4-Fluorphenyl)sulfonyl)-3-hydroxychinolin-2-carbonyl)glycin

### Beispiel 127

N-(6-Benzyloxy-3-hydroxychinolin-2-carbonyl)glycin

### Beispiel 128

N-(6-(4-Fluorbenzyloxy)-3-hydroxychinolin-2-carbonyl)glycin

### Beispiel 129

N-(7-Butyloxy-3-hydroxychinolin-2-carbonyl)glycin

### Beispiel 130

N-(7-Benzyloxy-3-hydroxychinolin-2-carbonyl)glycin

### Beispiel 131

N-(6-(cis-3-Hexenyl-1-oxy)-3-hydroxychinolin-2-carbonyl)glycin

### Beispiel 132

N-(6-(trans-3-Hexenyl-1-oxy)-3-hydroxychinolin-2-carbonyl)glycin

## Patentansprüche

1. Verbindungen der allgemeinen Formel l 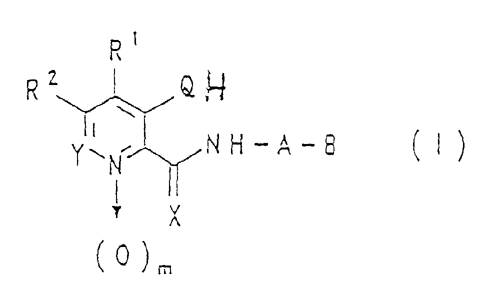 in welcher
Q O oder S,
X O,
Y C_{R}3,
m 0 und 1,
A (C₁-C₃)-Alkylen, das gegebenenfalls einfach substituiert ist mit Halogen, Cyano, trifluormethyl, (C₁-C₆)-Alkyl, (C₁-C₆)-Hydroxyalkyl, (C₁-C₆)-Alkoxy, -O-[CH2]_{X} C_{f}H_{(2f+1-g)}F_{g} oder
A -CHR^{S-} bedeutet, wobei R⁵ einen der Substituenten des a-C-Atoms einer a-Aminosäure bedeutet, insbesondere einer natürlichen L-Aminosäure und ihres D-Isomeren, wobei R⁵ nicht -CH₂-SH bedeutet,
B CO₂H,
R² Wasserstoff, (C₁-C₂₀-Alkyl, (C₂₋C₂₀)-Alkenyl, (C₂-C₂₀)-Alkinyl, (C₁-C₂₀)-Alkoxy, (C₂-C₂₀)-Alkenyloxy, (C₂-C₂ₒ)-Alkinyloxy, Retinyloxy, (C₁-C₂ₒ)-Alkoxy-(C₁-C₃)-alkyl, (C₂-C₂ₒ)-Alkenyloxy-(C₁-C₃)-alkyl, Retinyloxy-(C1-C3)-alkyl, (C₂-C₂₀)-Alkinyloxy-(C1-C₃)-alkyl, Halogen, Cyano, Trifluormethyl, (C₁-C₈)-Hydroxyalkyl, (C₁-C₂₀-Alkanoyl,(C₇-C₁₆)-Aralkanoyl, (C₆-C₁₂)-Aroyl, (C₆-C₁₂)-Aryl, (C₇-C₁₆)-Aralkyl, -O-[CH₂]ₓC_{f}H_{(2f+1-g})F_{g}, NR'R", (C₁-C₁₀)-Alkylmercapto, (C₁-C₁₀)-Alkylsulfinyl, (C₁-C₁₀)-Alkylsulfonyl, (C₆-C₁₂)-Arylmercapto, (C₆-C₁₂)-Arylsulfinyl), (C₆-C₁₂)-Arylsulfonyl, (C₇-C₁₂)-Aralkylmercapto, (C₇-C₁₂)-Aralkylsulfinyl, (C₇-C₁₂)-Aralkylsulfonyl, (C₆-C₁₂)-Aryloxy, (C₇-C₁₆)-Aralkyloxy, Carboxy, (C₁-C₂₀)-Alkoxycarbonyl, (C₁-C₁₂)-Alkoxy-(C₁-C₁₂)-alkoxycarbonyl, (C₆-C₁₂)- Aryloxycarbonyl, (C₇-C₁₆)-Aralkoxycarbonyl, (C₃-C₈)-Cycloalkoxycarbonyl, (C₂-C₂₀)-Alkenyloxycarbonyl, Retinyloxycarbonyl, (C₂-C₂₀)-Alklnyloxycarbonyl, (C₃ C₈)-Cycloalkyl-(C₁-C₅)-alkoxycarbonyl, (C₃-C₈)-Cycloalkoxy-(C₁-C₆)-alkoxycarbonyl, (C₆-C₁ )-Aryloxy-(C₁-C₆)-alkoxycarbonyl, (C₇-C₁₆)-Aralkoxy-(C₁-C₆)-alkoxycarbonyl,
Carbamoyl, N-(C₁-C₁₂)-Alkylcarbamoyl, N, N-Di-(C₁-C₁₂)-alkylcarbamoyl, N-(C₃-C₈)-Cycloalkylcarbamoyl, N,N-Dicyclo(C₃-C₈)-alkylcarbamoyl, N-(C₁-C₁₀)-Alkyl-N-(C₃-C₈)-cycloalkylcarbamoyl, N-(C₃-C₈)-Cycloalkyl-(C₁-C₆)-alkyl)carbamoyl, N-(C₁-C₆)-Alkyl-N-((C₃-C₈)-cycloalkyl-(C₁-C₆)-alkyl)carbamoyl, N-(+)-Dehydroa- bietylcarbamoyl, N-(C₁-C₆)-Alkyl-N-(+)-dehydroabietylcarbamoyl, N-(C₆-C₁₂)-Arylcarbamoyl, N-(C₇-C₁₆)-Aralkylcarbamoyl, N-(C₁-C₁₀)-Alkyl-N-(C₆-C₁₆)-arylcarbamoyl, N-(C₁-C₁₀)-Alkyl-N-(C₇-C₁₆)-aralkylcarba- moyl, N-((C₁-C₁₂)-Alkoxy-(C₁-C₁₀)-alkyl)carbamoyl, N-((C₆-C₁₆)-Aryloxy-(C₁-C₁₀)-alkyl)carbamoyl, N-((C₇-C₁₆)-Aralkyloxy-(C₁-C₁₀)-alkyl)carbamoyl,N-(C₁-C₁₀)-Alkyl-N-((C₁-C₁₀)-alkoxy-(C₇-C₁₀)-alkyl)carbamoyl, N-(C₁-C₁₀)-Alkyl-N-((C₆-C₁₂)-aryloxy-(C₁-C₁₀)-alkyl)carbamoyl, N-(C₁-C₁₀)-Alkyl-N-((C₇-C₁₆)-aralkyloxy-(C₁-C₁₀)-alkyl)carbamoyl, CON(CH₂)ₕ, worin eine CH₂-Gruppe durch O, S, N-(C₁-C₈)-alkylimino, N-(C₃-C₈)-Cycloalkylimino, N-(C₃-C₈)-Cycloalkyl-(C₁-C₄)-alkylimino, N-(C₆-C₁₂)-Arylimino, N-(C₇-C₁₆)-Aralkylimino oder N-(C₁-C₄)-Alkoxy-(C₁-C₆)-alkylimino ersetzt sein kann bedeutet,
wobei Aryl in der Weise substiuiert ist wie für R¹ und R³ definiert,
R¹ und R³ gleich oder verschieden sind und Wasserstoff, Halogen, (C₁-C₁₂)-Alkyl, (C₁-C₁₂)-Alkoxy, -O-[CH₂]ₓC₋ _{f}H_{(2f+1-g)}Hal_{g}, (C₁-C₁₂)-Alkoxy-(C₁-C₁₂)-alkyl, (C₁-C₈)-Alkoxy-(C₁-C₁₂)-alkoxy, (C₁-C₁₂)-Alkoxy-(C₁-C₈)-alkoxy-(C₂-C₆)-alkyl, (C₇-C₁₁)-Aralkyloxy, (C₃-C₈)-Cycloalkyl, (C₃-C₈)-Cycloalkyl-(C₁-C₈)-alkyl, (C₃-C₈)-Cycloalkyloxy, (C₃-C₈)-Cycloalkyl-(C₁-C₈)-alkoxy, (C₃-C₈)-Cycloalkyloxy-(C₁-C₈)-alkyl, (C₃-C₈)-Cycloalkyloxy-(C₁-C₈)-alkoxy, (C₃-C₈)-Cycloalkyl-(C₁-C₆)-alkyl-(C₁-C₆)-alkoxy, (C₃-C₈)-Cycloalkyl-(C₁-C₆)-alkoxy-(C₁-C₆)-alkyl, (C₃-C₈)-Cycloalkoxy-(C₁-C₆)-alkoxy-(C₁-C₆)-alkyl, NR^{Y}R^{Z}, (C₁-C₈)-Alkylmercapto, (C₁-C₈)-Alkylsulfinyl oder (C₁-C₈)-Alkylsulfonyl, (C₆-C₁₂)-Arylmercapto, (C₆-C₁₂)-Arylsulfinyl, (C₆-C₁₂)-Arylsulfonyl, (C₇-C₁₂)-Aralkylmercapto, (C₇-C₁₁)-Aralkylsulfinyl, (C₇-C₁₁ )-Aralkylsulfonyl, substituiertes (C₆-C₁₂)-Aryloxy-(C₁-C₆)-alkyl, (C₇-C₁₁)-Aralkoxy-(C₁-C₆)-alkyl, (C₆-C₁₂)-Aryloxy-(C₁-C₆)-alkoxy-(C₁-C₆)-alkyl, (C₇-C₁₁)-Aralkyloxy-(C₁-C₆)-alkoxy-(C₁-C₆)-alkyl, (C₆-C₁₂)-Aryloxy, (C₇-C₁₁)-Aralkyloxy, (C₆-C₁₂)-Aryloxy-(C₁-C₆)-alkoxy oder (C₇-C₁₁)-Aralkoxy-(C₁-C₆)-alkoxy bedeutet, wobei ein aromatischer Rest mit 1, 2, 3, 4 oder 5 gleichen oder verschiedenen Substituenten aus der Reihe Wasserstoff, Halogen, Cyano, Nitro, Trifluormethyl, (C₁-C₁₆)-Alkyl, (C₁-C₆)-Alkenyl, (C₁-C₆)-Hydroxyalkyl, (C₁-C₁₆)-Alkoxy, (C₁-C₁₆)-Alkenyloxy, -O-[CH₂]ₓC_{f}H_{(2f+1-g})F_{g}, -OCF₂CI, -O-CF₂-CHFCI, (C₁-C₆)-Alkylmercapto, (C₁-C₆)-Alkylsulfinyl, (C₁-C₆)-Alkylsulfonyl, (C₁-C₆)-Alkylcarbonyl, (C₁-C₆) (C₁-C₆)-Alkoxycarbonyl, Carbamoyl, N-(C₁-C₄)-Alkylcarbamoyl, N,N-Di-(C₁-C₄)-alkylcarbamoyl, (C₁-C₆)-Alkylcarbonyloxy (C₃-C₈)-Cycloalkylcarbamoyl, Phenyl, Benzyl, Phenoxy, Benzyloxy, NR^{Y}R^{Z}, Phenylmercapto, Phenylsulfonyl, Phenylsulfinyl, Sulfamoyl, N-(C₁-C₄)-Alkylsulfamoyl oder N,N-Di-(C₁-C₄)-alkylsulfamoyl trägt, oder gegebenenfalls bis zu 3 der vorstehend genannten gleichen oder verschiedenen Substituenten trägt und zwei benachbarte C-Atome des Aralkyloxyrestes gemeinsam eine Kette [CH₂-] und/oder -CH=CH-CH=CH- tragen, wobei eine CH₂₋Gruppe der Kette gegebenenfalls durch O, S, SO, S0₂ oder NR' ersetzt ist,
R² und R3
zusammen mit dem sie tragenden Pyridin einen Chinolin-Ring der Formel 1a bilden, 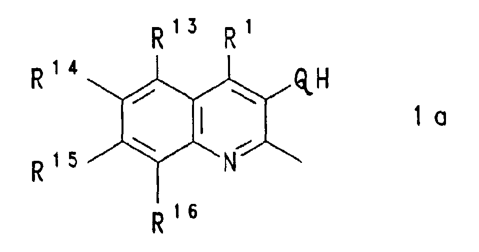 wobei
R¹³, R¹⁴, R¹⁵ und R¹⁶ Wasserstoff, (C₁-C₁₂)-Alkyl, (C₁-C₁₂)-Alkenyl; Chlor, Fluor, Brom, Trifluormethyl, (C₁-C₁₂)-Alkylsulfonyl, (C₁-C₁₂)-Alkylsulfinyl, Phenylsulfonyl, Phenylsulfinyl; wobei Phenyl gegebenenfalls mit Fluor, Chlor oder (C₁-C₅)-Alkoxy substituiert ist, (C₁-C₁₀)-Alk- oxy, -O-[CH₂]ₓ-C_{f}H_{(2f+1-g}), F_{g} oder einen Rest der Formel D bedeutet 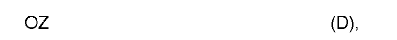
wobei Z für [CH2]ᵥ- [O]_{w}-[CH₂]ₜ-E steht,
worin E einen substituierten Phenylrest der Formel F 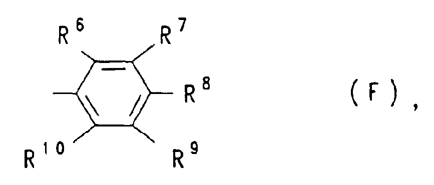 oder einen substituierten Heteroaryl-Rest oder einen substituierten (C₃-C₈)-Cycloalkylrest bedeutet,
wobei
v = 0, 1, 2, 3, 4, 5, 6 w = 0,1 und t = 0, 1, 2, 3, mit der Einschränkung, daß v ungleich 0 ist, falls w = 1 ist bedeutet und R⁶, R⁷, R⁸, R⁹ und R¹⁰ gleich oder verschieden sind und Wasserstoff, Halogen, Cyano, Nitro, Trifluormethyl, (C₁-C₆)-Alkyl, (C₃-C₈-Cycloalkyl, (C₁-C₆)-Alkoxy, -O-[CH₂₋]ₓC_{f}H_{(2f+1-g)}F_{g}, -OCF₂Cl, -O-CF₂-CHFCl, (C₁-C₆)-Alkylmercapto, (C₁-C₆)-Hydroxyalkyl, (C₁-C₆)-Alkoxy-(C₁-C₆)-alkoxy, (C₁-C₆)-Alkoxy-(C₁-C₆)-alkyl, (C₁-C₆)-Alkylsulfinyl, (C₁-C₆)-Alkylsulfonyl, (C₁-C₆)-Alkylcarbonyl, (C₁-C₈)-Alkoxycarbonyl, Carbamoyl, N-(C₁-C₈)-Alkylcarb- amoyl, N,N-Di-(C₁-C₈)-alkylcarbamoyl, gegebenenfalls mit Fluor, Chlor, Brom, Trifluormethyl und (C₁-C₆)-Alkoxy- substituiertes (C₇-C₁₁)-Aralkylcarbamoyl, N-(C₃-C₈)-Cycloalkylcarbamoyl, N-(C₃-C₈)-Cycloalkyl-(C₁-C₄)-alkyl- carbamoyl, (C₁-C₆)-Alkylcarbonyloxy, Phenyl, Benzyl, Phenoxy, Benzyloxy, NR'R", wie Amino, Anilino, N-Methylanilino, Phenylmercapto, Phenylsulfonyl, Phenylsulfinyl, Sulfamoyl, N-(C₁-C₈)-Alkylsulfamoyl oder N,N-Di-(C₁-C₈)-alkylsulfamoyl bedeuten, oder zwei benachbarte Substituenten gemeinsam eine Kette -[CH₂₋]ₙ oder -CH=CH-CH=CH- bedeuten, wobei eine CH₂-Gruppe der Kette gegebenenfalls durch O, S, SO, S0₂ oder NR' ersetzt ist, oder
falls R¹ und/oder R³ in der Bedeutung von (C₆-C₁₂)-Aryloxy, (C₇-C₁₁)-Aralkyloxy, (C₆-C₁₂)-Aryloxy-(C₁-C₆)-alkoxy, (C₇-C₁₁)-Aralkoxy -(C₁-C₆)-alkoxy oder einem entsprechenden endständige Cycloalkyl-Gruppen enthaltenden Rest stehen, so bedeutet dieser Rest vorzugsweise einen Rest der Formel D 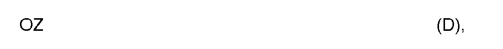
wobei Z für [CH₂]ᵥ- [O]_{w}-[CH₂]ₜ-E steht,
falls R¹ und/oder R³ in der Bedeutung von (C₇-C₁₁)-Aralkyl, (C₆-C₁₂)-Aryloxy-(C₁-C₆)-alkyl, (C₇-C₁₁)-Aralkoxy-(C₁-C₆)-alkyl oder einem entsprechenden endständige Cycloalkyl-Gruppen enthaltenden Rest stehen, so bedeutet dieser Rest vorzugsweise einen Rest der Formel Z, wobei Z in der obigen Bedeutung steht, aber v ≠ 0 ist, und
R^{Y} und R^{Z} gleich oder verschieden sind und Wasserstoff, (C₆-C₁₂)-Aryl, (C₁-C₁₀)-Alkyl, (C₃-C₁₀)-Cycloalkyl, (C₁-C₈)-Alkoxy-(C₁-C₈)-alkyl, (C₇-C₁₂)-Aralkoxy-(C₁-C₈)-alkyl, (C₆-C₁₂)-Aryloxy-(C₁-C₈)-alkyl,(C₁-C₁₀)-Alkanoyl, ggf. substituiertes (C₇-C₁₆)-Aralkanoyl, ggf substituiertes (C₆-C₁₂)-Aroyl bedeuten, oder
R^{Y} und R^{Z} gemeinsam für -[CH₂]ₕ₋ stehen, worin eine CH₂-Gruppe durch O, S, N-(C₁-C₄)-Alkanoylimino oder N-(C₁-C₄)-Alkoxycarbonylimino ersetzt sein kann, und
f 1 bis 8,
g 0, 1 bis (2f+1),
h 3 bis 6,
x 0 bis 3, und
n 3 oder 4 ist,
einschließlich der physiologisch wirksamen Salze, ausgenommen N-(3-Hydroxy-picolinoyl)-L-threonin, N-[(3-Hydroxy-6-methoxy-2-chinolinyl)-carbonyl]-D-Serin.

2. Verbindungen der Formel gemäß Anspruch 1, in der
Q O oder S,
X O,
Y C_{R}3,
m 0,
A eine (C₁-C₂)-Alkylengruppe,
B CO₂H,
R² Wasserstoff, Brom, Chlor, Cyano, (C₁-C₁₈)-Alkyl, (C₁-C₈)-Alkoxy, (C₁-C₁₈)-Alkoxymethyl, (C₂ C₁₈)-Alkenylo- xymethyl, (C₂-C₁₈)-Alkinyloxymethyl,Carbamoyl, N-(C₁-C₁₀)-Alkylcarbamoyl, N-((C₁-C₁₂)-Alkoxy-(C₁-C₄)-alkyl)carbamoyl, N, N-Di-(C₁-C₈)-alkylcarbamoyl, N-(C₃-C₈)-Cycloalkylcarbamoyl, N-(C₆-C₁₂)-Phenylcarbamoyl, N-(C₇-C₁₂)-Phenylalkylcarbamoyl, N-(C₁-C₆)-Alkyl-N-(C₆-C₁₂)phenylcarbamoyl, N-(C₁-C₆)-Alkyl-N-(C₇-C₁₂)-phenylalkylcarbamoyl, N-((C₁-C₆)-Alkoxy-(C₁-C₆)-alkyl)carbamoyl, Carboxy, (C₁-C₂₀) (C₁-C₂₀)-Alkoxycarbonyl, (C₂-C₂₀)-Alkenyloxycarbonyl, Retinyloxycarbonyl, (C₃-C₈)-Cycloalkoxycarbonyl, (C₃-C₈)-Cycloalkyl-(C₁-C₆)-alkoxycarbonyl, (C₃-C₈)-Cycloalkoxy-(C₁-C₆)-alkoxycarbonyl, Phenyl-(C₁-C₆)-alkoxycarbonyl, Phenoxy-(C₁-C₆)-alkoxycarbonyl, Benzyloxy-(C₁-C₆)-alkoxycarbonyl, wobei ein Phenylrest in der Weise substituiert ist wie für R¹ und R³ definiert und einer der Reste
R¹ oder R³ Wasserstoff und der andere einen Rest aus der Reihe Wasserstoff, Fluor, Chlor, (C₁-C₈)-Alkyl, (C₁-C₁₀)-Alkoxy, (C₅-C₆)-CyCloalkyl,(C₅-C₆)-CyCloalkyl-(C₁-C₆)-alkyl, (C₅-C₆)-CyCloalkyloxy, (C₅-C₆)-Cycloalkyl-(C₁-C₆)-alkoxy, (C₅-C₆)-Cycloalkyloxy-(C₁-C₆)-alkyl, (C₅-C₆)-Cycloalkyloxy-(C₅-C₆)-alk- oxy, (C₅-C₆)-Cycloalkyl-(C₁-C₄)-alkyl-(C₁-C₄)-alkoxy, (C₅-C₆)-Cycloalkyl-(C₁-C₄)-alkoxy-(C₁-C₂)-alkyl, (C₅-C₆)-Cycloalkoxy-(C₁-C₄)-alkoxy-(C₁-C₂)-alkyl, -O-[CH₂]ₓ-C_{f}H_{(2f+1-g)}F_{g}, (C₁-C₆)-Alkoxy-(C₁-C₆)-alkyl, (C₁-C₆)-Alkoxy-(C₁-C₆)-alkoxy, (C₁₋C₆)-Alkoxy-(C₁-C₄)-alkoxy-(C₁-C₂)-alkyl, substituiertes (C₆-C₁₂)-Phenoxy, (C₇-C₁₁)-Phenylalkyloxy, (C₆-C₁₂)-Phenoxy-(C₁-C₆)-alkoxy oder (C₇-C₁₁)-Phenylalkoxy-(C₁-C₆)-alkoxy, Phenoxy-(C₁-C₄)-alkyl, (C₇-C₁₁)-Phenylalkyloxy-(C₁-C₄)-alkyl, Phenoxy-(C₁-C₄)-alkoxy-(C₁-C₂)-alkyl, (C₇-C₁₁)-Phenylalkyloxy-(C₁-C₄)-alkoxy-(C₁-C₂)-alkyl bedeutet, wobei ein aromatischer Rest mit 1, 2 oder 3 gleichen oder verschiedenen Substituenten aus der Reihe Fluor, Chlor, Cyano, Trifluormethyl, (C₁-C₁₂)-Alkyl, (C₂-C₁₂)-Alkenyl, (C₂-C₁₂)-Alkenyloxy, (C₁-C₁₂)-Alkoxy, substituiert ist und
falls R¹ oder R³ in der Bedeutung von (C₆-C₁₂)-Phenoxy, (C₇-C₁₁)-Phenylalkyloxy, (C₆-C₁₂)-Phenoxy-(C₁-C₆)-alkoxy, (C₇-C₁₁)-Phenylalkoxy-(C₁-C₆)-alkoxy, (C₅-C₆)-Cycloalkyloxy, (C₅-C₆)-Cycloalkyl-(C₁-C₆)-alkoxy, (C₅-C₆)-Cycloalkoxy-(C₁-C₆)-alkoxy oder (C₅-C₆)-Cycloalkyl-(C₁-C₄)-alkyl-(C₁-C₄)-alkoxy steht, dieser Rest im speziellen einen Rest der Formel D bedeutet 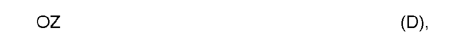
in der Z -[CH2]ᵥ-[O]_{w}-[CH₂]t-E, bedeutet,
wobei E einen substituierten Phenylrest der Formel F 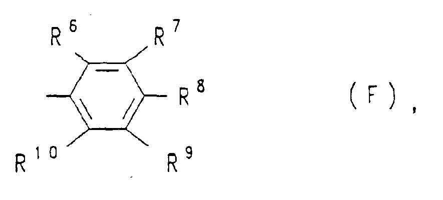
oder einen (C₃-C₈)-Cycloalkylrest bedeutet, wobei
v = 0, 1, 2, 3, w = 0, 1 und t = 0,1 sein kann, mit der Einschränkung, daß v ungleich 0 ist, falls w = 1 ist, und worin R⁶, R⁷, R⁸, R⁹ und R¹⁰ gleich oder verschieden sind und Wasserstoff, Fluor, Chlor, Cyano, Trifluormethyl, (C₁-C₆)-Alkyl, (C₁-C₆)-Alkoxy, -O-[CH₂₋]ₓC_{f}H_{(2f+1-g)}F_{g}, N-(C₁-C₈)-Alkylcarbamoyl, N,N-Di-(C₁-C₈)-alkylcarbamoyl, N-(C₃-C₈)-Cycloalkylcarbamoyl, gegebenenfalls mit Fluor, Chlor, Trifluormethyl und (C₁-C₆)-Alkoxy substituiertes (C₇₋C₁₁)-Phenylalkylcarbamoyl bedeuten, oder
falls R¹ oder R³ in der Bedeutung von Phenyl, Phenoxy-(C₁-C₆)-alkyl; (C₇-C₁₁)-Phenylalkyl, (C₇-C₁₁)-Phenylalky- loxy-(C₁-C₄)-alkyl, (C₅-C₆)-Cycloalkyl, (C₅-C₆)-Cycloalkyl-(C₁-C₆)-alkyl, (C₅-C₆)-Cycloalkoxy-(C₁-C₄)-alkyl, (C₅-C₆)-Cycloalkyl-(C₁-C₄)-alkoxy-(C₁-C₂)-alkyl, (C₅-C₆)-Cycloalkoxy-(C₁-C₄)-alkoxy-(C₁-C₂)-alkyl, steht, dieser Rest im speziellen einen Rest der Formel Z bedeutet,
worin
v = 1, 2, 3 und 4, w = 0 und t = 0 oder
v = 1,2, 3 und 4, w = 1 und t = 0 oder
v = 1, 2, 3 und 4, w = 1, t = 1
sind.

3. Verbindungen der Formel I gemäß Anspruch 1, in der
Q O, S, bevorzugt O,
X O,
Y CR³,
m 0,
A eine -CH₂-Gruppe,
B -C0₂H,
R¹ Wasserstoff,
R² und R³ zusammen mit dem sie tragenden Pyridin einen Chinolinring bilden, wobei
R¹³, R¹⁵ und R¹⁶ Wasserstoff,
R¹⁴ Wasserstoff, (C₁-C₁₂)-Alkyl, (C₁-C₁₂)-Alkenyl, Chlor, Fluor, Brom, Trifluormethyl, (C₁-C₁₂)-Alkylsulfonyl, (C₁-C₁₂)-Alkylsulfinyl, Phenylsulfonyl, Phenylsulfinyl; wobei Phenyl gegebenenfalls einfach mit Fluor, Chlor oder (C₁-C₅)-Alkoxy substituiert ist, (C₁-C₁₀)-Alkoxy, -O-[CH₂]X-Cf^{H}(2f+i-g), Fg, Benzyloxy, im Phenylring gegebenenfalls einfach substituiert ist mit Fluor, Chlor oder (C₁-C₅)-Alkoxy bedeutet, einschließlich der physiologisch wirksamen Salze.

4. Verbindungen der Formel I gemäß den Ansprüchen 1 und 2, in der
Q O,
X O,
Y CR³,
m 0,
A eine -CH₂-Gruppe,
B C0₂H,
R¹ Wasserstoff, (C₁-C₁₀)-Alkoxy, (C₅-C₆)-Cycloalkyloxy, (C₅-C₆)-Cycloaykyl-(C₁-C₂)-alkoxy; -O-[CH2]_{X} Cf^{H}(2f+1-g)^{F}g (C₁-C₄)-Alkoxy-(C₁-C₄)-alkoxy, substituiertes Phenoxy, substituiertes Benzyloxy bedeutet, wobei der Phenylrest mit einem Substituenten aus der Reihe Fluor, Chlor, Cyano, Trifluormethyl, (C₁-C₄)-Alkyl oder (C₁-C₄)-Alkoxy substituiert ist und
R² und R³ Wasserstoff bedeuten, einschließlich der physiologisch wirksamen Salze.

5. Verbindung der Formel I gemäß den Ansprüchen 1 und 2, in der
Q S,
X O.
Y C_{R}3,
m 0,
A eine -CH₂-Gruppe bedeutet,
B -C0₂H,
R¹ Wasserstoff und
R² und R³ Wasserstoff bedeuten.

6. Verbindung der Formel I gemäß den Ansprüchen 1 und 3, in der
Q O,
X O,
Y CR3,
m 0,
A eine -CH₂-Gruppe bedeutet,
B -CO₂H,
R¹ Wasserstoff und
R² und R³ zusammen mit dem sie tragenden Pyridin einen Chinolin-Ring bilden.

7. Verbindungen der Formel gemäß den Ansprüchen 1 bis 6 zuzüglich 3-Hydroxypyridin-2carbonsäure-N-(carboxymethyl)amid, N-(3-Hydroxypicolinyl)-L-threonin und N-[(3-Hydroxy-6-methoxy-2-chinolinyl)-carbonyl]-D-serin zur Anwendung als Arzneimittel.

8. Verbindungen gemäß den Ansprüchen 1 bis 7 für die Anwendung zur Hemmung der Kollagenbiosynthese, ein Schließlich Verbindungen der Formel in denen R⁵ -CH₂-SH bedeutet.

9. Verbindungen gemäß den Ansprüchen 1 bis 7 als Inhibitoren der Prolylhydroxylase, ein Schließlich Verbindungen der Formel I, in denen R⁵ -CH₂-SH bedeutet.

10. Verbindungen gemäß den Ansprüchen 1 bis 7 zur Anwendung als Fibrosupressiva, ein Schließlich Verbindungen der Formel I, in denen R⁵ -CH₂-SH bedeutet.

11. Verbindungen gemäß den Ansprüchen 1 bis 7 zur Herstellung eines Arzneimittels gegen fibrotische Erkrankungen, ein Schließlich Verbindungen der Formel I, in denen R⁵ -CH₂-SH bedeutet.

12. Verbindungen gemäß den Ansprüchen 1 bis 7 zur Herstellung eines Arzneimittels gegen fibrotische Erkrankungen der Leber ein Schließlich Verbindungen der Formel I, in denen R⁵ -CH₂-SH bedeutet.

13. Verbindungen gemäß den Ansprüchen 1 bis 7 zur Herstellung eines Arzneimittels gegen fibrotische Erkrankungen der Lunge, ein Schließlich Verbindungen der Formel I, in denen R⁵ -CH₂-SH bedeutet.

14. Verbindungen gemäß den Ansprüchen 1 bis 7 zur Herstellung eines Arzneimittels gegen fibrotische Erkrankungen der Haut, ein Schließlich Verbindungen der Formel I, in denen R⁵ -CH₂-SH bedeutet.

15. Prodrugs der Verbindungen der Formel I gemäß den Ansprüchen 1 bis 7.

16. Verfahren zur Herstellung von Verbindungen nach Formel I gemäß den Ansprüchen 1 bis 7, in der A einen substituierten Alkylen-Teil, B = CO₂H, Y = CR3 und m = und 1 bedeuten, indem
i1.) Pyridin-2-carbonsäuren der Formel II (R¹¹ = H) mit den Aminoestern der Formel III zu den Amidestern der Formel IV umgesetzt werden, oder
i2.) Pyridin-2-carbonsäureester der Formel II (R¹¹ = niedrig Alkyl) unter den Bedingungen der Aminolyse zu den Verbindungen der Formel IV umgesetzt werden; 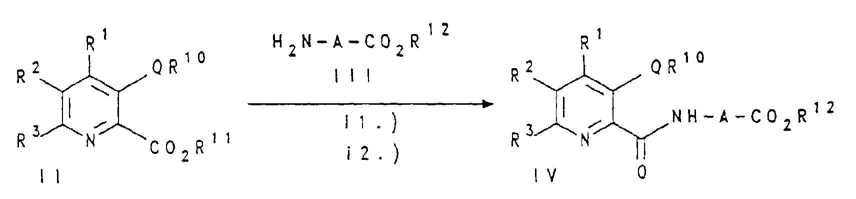 und
ii) die Verbindungen der Formeln I und V aus ihren Estern der Formel IV freigesetzt werden; 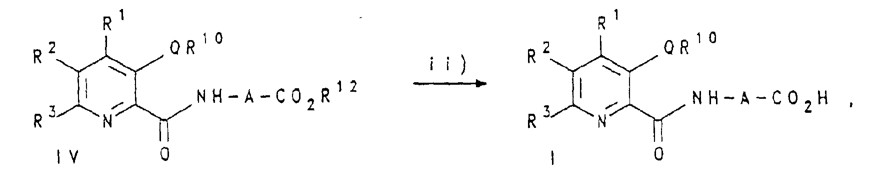 und wobei ggf.
iii) die Verbindungen der Formeln IV durch Alkylierung von Verbindungen der Formel V hergestellt sind, 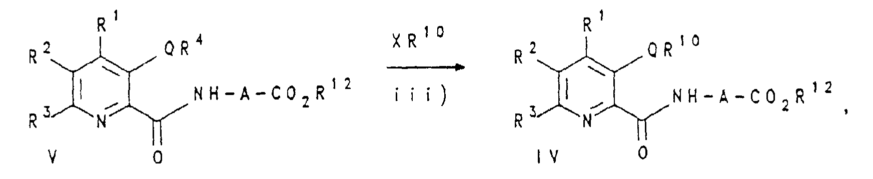 wobei X für eine Abgangsgruppe, insbesondere für Halogen, OSO₂Me, OSO₂ Phenyl, steht und ggf.
iv) die Verbindungen der Formeln I, V oder IV, in ihre Pyridin-N-oxide (I' oder VI) 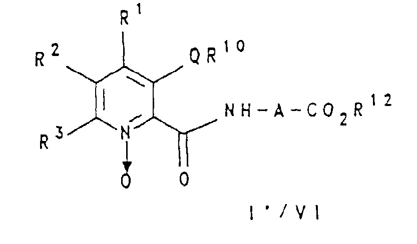 übergeführt werden.

## Claims

1. A compound of the formula I 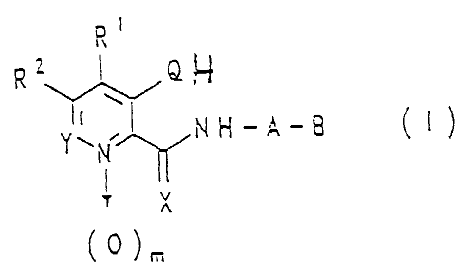 in which
Q is O or S,
X is O,
Y is CR3,
m is 0 and 1.
A is (C₁-C₃)-alkylene, which is optionally substituted once by halogen, cyano, trifluoromethyl, (C₁-C₆)-alkyl, (C₁-C₆)-hydroxyalkyl, (C₁-C₆)-alkoxy or -O-[CH₂]_{X}-C_{f}H(2f+1-g)Fg, or
A is -CHR^{S-}, where R⁵ is one of the substituents of the a-carbon atom of an a-amino acid, in particular of a natural L-amino acid and of its D-isomer, R⁵ not being -CH₂-SH,
B is CO₂H,
R² is hydrogen, (C₁-C₂₀)-alkyl, (C₂-C₂₀)-alkenyl, (C₂-C₂₀)-alkynyl, (C₁-C₂₀)-alkoxy, (C₂-C₂ₒ)-alkenyloxy, (C₂-C₂ₒ)-alkynyloxy, retinyloxy, (C₁-C₂0)-alkoxy-(C₁-C₃)-alkyl, (C₂-C₂₀)-alkenyloxy-(C₁-C₃)-alkyl, retinyloxy-(C₁-C₃)-alkyl, (C₂-C₂₀)-alkynyloxy-(C₁-C₃)-alkyl, halogen, cyano, trifluoromethyl, (C₁-C₈)-hydroxyalkyl, (C₁-C₂₀₎- alkanoyl, (C₁-C₆)-aralkanoyl, (C₆-C₁₂)-aroyl, (C₆-C₁₂)-aryl, (C₇-C₁₆)-aralkyl, -O-[CH2]_{X} Cf^{H}(2f+1-g)^{F}g NR'R", (C₁-C₁₀)-alkylmercapto, (C₁-C₁₀)-alkylsulfinyl, (C₁-C₁₀)-alkylsulfonyl, (C₆-C₁₂)-arylmercapto, (C₆-C₁₂)-arylsulfinyl, (C₆-C₁₂)-arylsulfonyl, (C₇-C₁₂)-aralkylmercapto, (C₇-C₁₂)-aralkylsulfinyl, (C₇-C₁₂)-aralkylsulfonyl, (C₆-C₂)-aryloxy, (C₇-C₁₆)-aralkyloxy, carboxyl, (C₁-C₂₀)-alkoxycarbonyl, (C₁-C₁₂)-alkoxy-(C₁-C₁₂)-alkoxycarbonyl, (C₆-C₁₂)-aryloxycarbonyl, (C₇-C₁₆)-aralkoxycarbonyl, (C₃-C₈)-cycloalkoxycarbonyl, (C₂-C₂₀)-alkenyloxycarbonyl, retinyloxycarbonyl, (C₂-C₂ₒ)-alkynyloxycarbonyl, (C₃-C₈)-cycloalkyl-(C₁-C₆)-alkoxycarbonyl, (C₃-C₈)-cycloalkoxy-(C₁-C₆)-alkoxycarbonyl, (C₆-C₆)-aryloxy-(C₁-C₆)-alkoxycarbonyl, (C₇-C₁₆)-aralkoxy-(C₁-C₆)-alkoxycarbonyl,
carbamoyl, N-(C₁-C₁₂)-alkylcarbamoyl, N,N-di-(C₁-C₁₂)-alkylcarbamoyl, N-(C₃-C₈)-cycloalkylcarbamoyl, N, N-dicyclo-(C₃-C₈)-alkylcarbamoyl, N-(C₁-C₈)-alkyl-N-(C₃-C₈)-cycloalkylcarbamoyl_{;} N-(C₃-C₈)-cycloalkyl-(C₁-C₆)-alkylcarbamoyl, N-(C₁-C₆)-alkyl-N-((C₃-C₆)-cycloalkyl-(C₁-C₆)-alkyl)carbamoyl, N-(+)-dehydroabie- tylcarbamoyl, N-(C₁-C₆)-alkyl-N-(+)-dehydroabietylcarbamoyl,N-(C₆-C₂)-arylcarbamoyl, N-(C₇-C₁₆)-ar- alkylcarbamoyl, N-(C₁-C₁₀)-alkyl-N-(C₆-Cᵢ₆)arylcarbamoyl, N-(C₁-C₁₆)-alkyl-N-(C₇-C₁₆)-aralkylcarbamoyl, N-((C₁-C₁₂)-alkoxy-(C₁-C₁₀)alkyl)carbamoyl, N-((C₆-Cᵢ₆)-aryloxy-(Cᵢ-Cᵢₒ)alkyl)carbamoyl, N-((C₇-C₁₆)-aralkyloxy-(C₁-C₁₀)alkyl)carbamoyl, N-(C₁-C₁₀)-alkyl-N-((C₁-C₁₀)-alkoxy-(C₇-C₁₀)alkyl)carbamoyl, N-(C₁-C₁₀)-alkyl-N-((C₆-C₁₂)-aryloxy(C₁-C₁₀)alkyl)-carbamoyl, N-(C₁-C₁₆)-alkyl-N-((C₁-C₁₆)-aralkyloxy-(C₁-C₁₀) alkyl)carbamoyl or CON(CH₂)ₕ in which a CH₂ group can be replaced by O, S, N-(C₁-C₈)-alkylimino, N-(C₃-C₈)-cycloalkylimino, N-(C₃-C₈)-cycloalkyl-(C₁-C₄)-alkylimino, N-(C₆-C₁₂)-arylimino, N-(C₇-C₁₆)-aralkylimino or N-(C₁-C₄)-alkoxy-(C₁-C₆)-alkylimino,
where aryl is substituted in the manner defined for R¹ and R³,
R¹ and R³ are identical or different and are hydrogen, halogen, (C₁-C₁₂)-alkyl, (C₁-C₁₂)-alkoxy, -O-[CH₂]x-C_{f}H_{(2r+1-g)}Halg, (C₁-C₁₂)-alkoxy-(C₁-C₁₂)-alkyl, (C₁-C₈)-alkoxy-(C₁-C₁₂)-alkoxy, (C₁-C₁₂)-alkoxy-(C₁-C₈)-alkoxy-(C₂-C₆)-alkyl, (C₇-C₁₁)-aralkyloxy, (C₃-C₈)-cycloalkyl, (C₃-C₈)-cycloalkyl-(C₁-C₈)-alkyl, (C₃-Cₛ)-cycloalkyloxy, (C₃-C_{S})-cycloalkyl-(C₁-C₈)-alkoxy, (C₃-C₈)-cycloalkyloxy-(C₁-C₈)-alkyl, (C₃-C₈)-cycloalkyloxy-(C₁-C₈)-alkoxy, (C₃-C₈)-cycloalkyl-(C₁-C₆)-alkyl-(C₁-C₆)-alkoxy, (C₃-C₈)-cycloalkyl-(C₁-C6)-alkoxy-(C₁-C₆)-alkyl, (C₃-C₈)-cycloalkoxy-(C₁-C₆)-alkoxy-(C₁-C₆)-alkyl, NR^{Y}R^{Z}, (C₁-C₈)-alkylmercapto, (C₁-C₈)-alkylsulfinyl or (C₁-C₈)-alkylsulfonyl, (C₆-C₁₂)-arylmercapto, (C₆-C₁₂)-arylsulfinyl, (C₆-C₁₂)-arylsulfonyl, (C₇-C₁₂)-aralkylmercapto, (C₇-C₁₁)-aralkylsulfinyl; (C₇-C₁₁)-aralkylsulfonyl, substituted (C₆-C₁₂)-aryloxy-(C₁-C₆)-alkyl, (C₇-C₁₁)-aralkoxy-(C₁-C₆)-alkyl, (C₆-C₁₂)-aryloxy-(C₁-C₆)-alkoxy-(C₁-C₆)-alkyl, (C₇-C₁₁)-aralkyloxy-(C₁-C₆)-alkoxy-(C₇-C₆)-alkyl, (C₆-C₁₂)-aryloxy, (C₇-C₁₁)-aralkyloxy, (C₆-C₁₂)-aryloxy-(C₁-C₆)-alkoxy or (C₇-C₁₁)-aralkoxy-(C₁-C₆)-alkoxy, where an aromatic radical carries is substituted by 1, 2, 3, 4 or 5 identical or different substituents from the group hydrogen, halogen, cyano, nitro, trifluoromethyl, (C₁-C₁₆)-alkyl, (C₁-C₁₆)-alkenyl, (C₁-C₆)-hydroxyalkyl, (C₁-C₁₆)-alkoxy, (C₁-C₁₆)-alkenyloxy, -O-[CH₂]ₓ-C_{f}H_{(2f+1-g)}Fg, -OCF₂CI, -0-CF₂₋CHFCI, (C₁-C₆)-alkylmercapto, (C₁-C₆)-alkylsulfinyl, (C₁-C₆)-alkylsulfonyl; (C₁-C₆)-alkylcarbonyl, (C₁-C₆)-alkoxycarbonyl, carbamoyl, N-(C₁-C₄)-alkylcarbamoyl, N,N-di-(C₁-C₄)-alkylcarbamoyl, (C₁-C₆)-alkylcarbonyloxy, (C₃-C₈)-cycloalkylcarbamoyl, phenyl, benzyl, phenoxy, benzyloxy, NR^{Y}R^{Z}, phenylmercapto, phenylsulfonyl, phenylsulfinyl, sulfamoyl, N-(C₁-C₄)-alkylsulfamoyl or N,N-di-(C₁-C₄)-alkylsulfamoyl, or optionally carries up to 3 of the abovementioned identical or different substituents, and two adjacent carbon atoms of the aralkyloxy radical together carry a chain -[CH₂-] and/or -CH=CH-CH=CH-, where a CH₂ group of the chain is optionally replaced by O, S, SO, S0₂ or NR',
R² and R³ form, together with the pyridine carrying them, a quinoline ring of the formula 1 a, 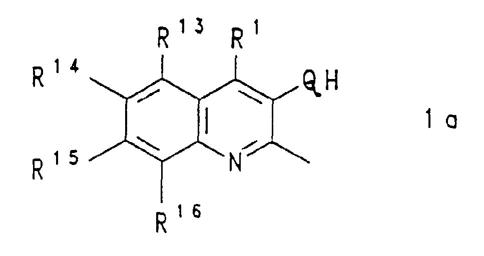 where
R¹³, R¹⁴, R¹⁵ and R¹⁶ are hydrogen, (C₁-C₁₂)-alkyl, (C₁-C₁₂)-alkenyl, chlorine, fluorine, bromine, trifluoromethyl, (C₁-C₁₂)-alkylsulfonyl, (C₁-C₁₂)-alkylsulfinyl, phenylsulfonyl, phenylsulfinyl; where phenyl is optionally substituted by fluorine, chlorine or (C₁-C₅)-alkoxy, (C₁-C₁₀)-alkoxy, -O-[CH₂]ₓ-C_{f}H_{(2f+1-g)} Fg, or a radical of the formula D 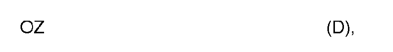
where Z is [CH_{2]v}-[O]_{w}-[CH₂]ₜ-E,
in which E is a substituted phenyl radical of the formula F 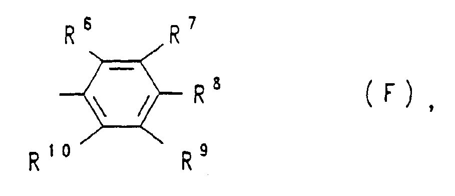
or a substituted heteroaryl radical or a substituted (C₃-C₈)-cycloalkyl radical
where
v is 0, 1, 2, 3, 4, 5 or 6, w is 0 or 1, and t is 0, 1, 2 or 3, with the restriction that v is not 0 if w is 1, and R⁶, R⁷, R⁸, R⁹ and R¹⁰ are identical or different and are hydrogen, halogen, cyano, nitro, trifluoromethyl, (C₁-C₆)-alkyl, (C₃-C₈)-cycloalkyl, (C₁-C₆)-alkoxy, -O-[CH₂]ₓ-C_{f}H_{(2f+1-g)}F_{g}, -OCF₂CI, -0-CF₂-CHFCI, (C₁-C₆)-alkylmercapto, (C₁-C₆)-hydroxyalkyl, (C₁-C₆)-alkoxy-(Cᵢ-C₆)-alkoxy, (C₁-C₆)-alkoxy-(C₁-C₆)-alkyl, (C₁-C₆)-alkylsulfinyl, (C₁-C₆)-alkylsulfonyl, (C₁-C₆)-alkylcarbonyl, (C₁-C₈)-alkoxycarbonyl, carbamoyl, N-(C₁-C₈)-alkylcarbamoyl, N,N-di-(C₁-C₈)-alkylcarbamoyl, (C₇-C₁₁)-aralkylcarbamoyl which is optionally substituted by fluorine, chlorine, bromine, trifluoromethyl or (C₁-C₆)-alkoxy, N-(C₃-C₈)-cycloalkylcarbamoyl, N-(C₃-C₈)-cycloalkyl-(C₁-C₄)-alkylcarbamoyl, (C₁-C₆)-alkylcarbonyloxy, phenyl, benzyl, phenoxy, benzyloxy, NR'R", such as amino, anilino, N-methylanilino, phenylmercapto, phenylsulfonyl, phenylsulfinyl, sulfamoyl, N-(C₁-C₈)-alkylsulfamoyl or N,N-di-(C₁-C₈)-alkylsulfamoyl, or two adjacent substituents together are a chain -[CH₂]ₙ, or -CH=CH-CH=CH-, where a CH₂ group of the chain is optionally replaced by O, S, SO, S0₂ or NR', or
if R¹ and/or R³ have the meaning of (C₆-C₁₂)-aryloxy, (C₇-C₁₁)-aralkyloxy, (C₆-C₁₂)-aryloxy-(Ci-C6)-alkoxy, (C₇-C₁₁)-aralkoxy-(C₁-C₆)-alkoxy or a corresponding radical containing terminal cycloalkyl groups, this radical is then preferably a radical of the formula D 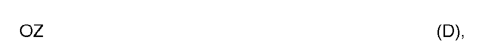
where Z is [CH₂]ᵥ-[O]_{w}-[CH₂]ₜ-E,
if R¹ and/or R³ have the meaning of (C₇-C₁₁)-aralkyl, (C₆-C₁₂)-aryloxy-(C₁-C₆)-alkyl, (C₇-C₁₁)-aralkoxy-(C₁-C₆)-alkyl or a corresponding radical containing terminal cycloalkyl groups, this radical is then preferably a radical of the formula Z, where Z has the above meaning, but v is not 0, and
R^{Y} and R^{Z} are identical or different and are hydrogen, (C₆-C₁₂)-aryl, (C₁-C₁₀)-alkyl, (C₃-C₁₀)-cycloalkyl, (C₁-C₈)-alkoxy-(C₁-C₈)-alkyl, (C₇-C₁₂)-aralkoxy-(C₁-C₈)-alkyl, (C₆-C₁₂)-aryloxy-(C₆-C₁₂)-alkyl, (C₁-C₁₀)-alkanoyl, optionally substituted (C₇-C₁₆)-aralkanoyl or optionally substituted (C₆-C₁₂)-aroyl; or
R^{Y} and R^{Z} together are -[CH₂]ₕ- in which a CH₂ group can be replaced by O, S, N-(C₁-C₄)-alkanoylimino or N-(C₁-C₄)-alkoxycarbonylimino, and
f is 1 to 8,
g is 0 or 1 to (2f+1),
h is 3 to 6,
x is 0 to 3, and
n is 3 or 4,
including the physiologically active salts, with the exception of N-(3-hydroxypicolinoyl)-L-threonine and N-[(3-hydroxy-6-methoxy-2-quinolinyl)-carbonyl]-D-serine.

2. A compound of the formula I as claimed in claim 1, in which
Q is 0 or S,
X is O,
Y is CR³,
m is 0.
A is a (C₁-C₂)-alkylene group,
B is C0₂H,
R² is hydrogen, bromine, chlorine, cyano, (C₁-C₁₈)-alkyl, (C₁-C₈)-alkoxy, (C₁-C₁₈)-alkoxymethyl, (C₂-C₁₈)-alkenyloxymethyl, (C₂-C₁₈)-alkynyloxymethyl, carbamoyl, N-(C₁-C₁₀)-alkylcarbamoyl, N-((C₁-C₁₂)-alkoxy-(C₁-C₄)-alkyl)carbamoyl, N,N-di-(C₁-C₈)-alkylcarbamoyl, N-(C₃-C₈)-cycloalkylcar- bamoyl, N-(C₆-C₁₂)-phenylcarbamoyl,N-(C₇-C₁₂)-phenylalkyl-carbamoyl, N-(C₁-C₆)-alkyl-N-(C₆-C₁₂)-phenylcarbamoyl, N-(C₁-C₆)-alkyl-N-(C₇-C₁₂)-phenylalkylcarbamoyl, N-((C₁-C₆)-alkoxy-(C₁-C₆) alkyl)-carbamoyl, carboxyl, (C₁-C₂₀)-alkoxycarbonyl, (C₂-C₂ₒ)-alkenyloxycarbonyl, retinyloxycarbonyl, (C₃-C₈)-cycloalkoxycarbonyl, (C₃-C₈)-cycloalkyl-(C₁-C₆)-alkoxycarbonyl, (C₃-C₈)-cycloalkoxy-(C₁-C₆)-alkoxycarbonyl,phenyl-(C₁-C₆)-alkoxycarbonyl, phenoxy-(C₁-C₆)-alkoxycarbonyl or benzyloxy-(C₁-C₆)-alkoxycarbonyl, where a phenyl radical is substituted in the manner defined for R¹ and R³, and one of the radicals
R¹ or R³ is hydrogen and the other a radical from the group hydrogen, fluorine, chlorine, (C₁-C₈)-alkyl, (C₁-Cᵢₒ)-alkoxy, (C₅-C₆)-cycloalkyl, (C₅-C₆)-cycloalkyl-(C₁-C₆)-alkyl; (C₅-C₆)-cycloalkyloxy, (C₅-C₆)-cycloalkyl-(C₁-C₆)-alkoxy, (C₅-C₆)-cycloalkyloxy-(C₁-C₆)-alkyl, (C₅-C₆)-cycloalkyloxy-(C₁-C₆)-alkoxy, (C₅-C₆)-cycloalkyl-(C₁-C₄)-alkyl-(C₁-C₄)-alkoxy, (C₅-C₆)-cycloalkyl-(C₁-C₄)-alkoxy-(C₁-C₂)-alkyl, (C₅-C₆)-cycloalkoxy-(C₁-C₄)-alkoxy-(C₁-C₂)-alkyl, -O-[CH₂]x-C_{f}H_{(2f+1-g})Fg, (C₁-C₆)-alkoxy-(C₁-C₆)-alkyl, (C₁-C₆)-alkoxy-(C₁-C₆)-alkoxy, (C₁-C₆)-alkoxy-(C₁-C₄)-alkoxy-(C₁-C₂)-alkyl; substituted (C₆-C₁₂)-phenoxy, (C₇-C₁₁)-phenylalkyloxy, (C₆-C₁₂)-phenoxy-(C₁-C₆)-alkoxy or (C₇-C₁₁)-phenylalkoxy-(C₁-C₆)-alkoxy, phenoxy-(C₁-C₄)-alkyl, (C₁-C₄)-phenylalkyloxy-(C₁-C₄)-alkyl,phenoxy-(C₁-C₄)-alkoxy-(C₁-C₂)-alkyl or (C₇-C₁₁)-phenylalkyloxy-(C₁-C4)-alkoxy-(C₁-C₂)-alkyl, where an aromatic radical is substituted by 1, 2 or 3 identical or different substituents from the group fluorine, chlorine, cyano, trifluoromethyl, (C₁-C₁₂)-alkyl, (C₂-C₁₂)-alkenyl, (C₂-Cᵢ₂)-alkenyloxy or (C₁-C₁₂)-alkoxy,
and
if R¹ or R³ has the meaning of (C₆-C12)-phenoxy, (C₇-C₁₁)-phenylalkyloxy, (C₆-C₁₂)-phenoxy-(Ci-C6)-alkoxy, (C₇-C₁₁)-phenylalkoxy-(C₁-C₆)-alkoxy, (C₅-C₆)-cycloalkyloxy, (C₅-C₆)-cycloalkyl-(C₁-C₆)-alkoxy, (C₅-C₆)-cycloalkoxy-(C₁-C₆)-alkoxy or (C₅-C₆)-cycloalkyl-(C₁-C₄)-alkyl-(C₁-C₄)-alkoxy, this radical is then, especially, a radical of the formula D 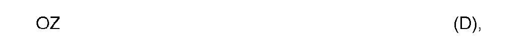 in which Z is -[CH₂]ᵥ-[O]_{w}-[CH₂]ₜ-E,
where E is a substituted phenyl radical of the formula F 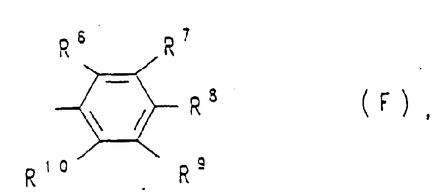 or a (C₃-C₈)-cycloalkyl radical, where
v is 0, 1, 2 or 3, w is 0 or 1, and t can be 0 or 1, with the restriction that v is not 0 if w is 1, and in which R⁶, R⁷, R⁸, R⁹ and R¹⁰ are identical or different and are hydrogen, fluorine, chlorine, cyano, trifluoromethyl, (C₁-C₆)-alkyl, (C₁-C₆)-alkoxy, -O-[CH₂]x-C_{f}H_{(2f+1-g)}F_{g}, N-(C₁-C₈)-alkylcarbamoyl, N,N-di-(C₁-C₈)-alkylcarbamoyl, N-(C₃-C₈)-cy- cloalkylcarbamoyl or (C₇-C₁₁)-phenylalkylcarbamoyl which is optionally substituted by fluorine, chlorine, trifluoromethyl or (C₁-C₆)-alkoxy, or
if R¹ or R³ have the meaning of phenyl, phenoxy-(C₁-C₆)-alkyl, (C₇-C₁₁)-phenylalkyl, (C₇-C₁₁)-phenylalkyloxy-(C₁-C₄)-alkyl, (C₅-C₆)-cycloalkyl, (C₅-C₆)-cycloalkyl-(C₁-C₆)-alkyl, (C₅-C₆)-cycloalkoxy-(C₁-C₄)-alkyl, (C₅-C₆)-cycloalkyl-(C₁-C₄)-alkoxy-(C₁-C₂)-alkyl or (C₅-C₆)-cycloalkoxy-(C₁-C₄)-alkoxy-(C₁-C₂)-alkyl; this radical is then, especially, a radical of formula Z
in which
v is 1, 2, 3 and 4, w is 0 and t is 0, or
v is 1, 2, 3 and 4, w is 1 and t is 0, or
v is 1, 2, 3 and 4, w is 1 and t is 1.

3. A compound of the formula I as claimed in claim 1, in which
Q is O or S, preferably O,
X is O.
Y is CR³.
m is 0,
A is a -CH₂- group,
B is -C0₂H,
R¹ is hydrogen,
R² and R³, together with the pyridine carrying them, form a quinoline ring, where
_{R}13, R¹⁵ and R¹⁶ are hydrogen,
R¹⁴ is hydrogen, (C₁-C₁₂)-alkyl, (C₁-C₁₂)-alkenyl, chlorine, fluorine, bromine, trifluoromethyl, (C₁-C₁₂)-alkylsulfonyl, (C₁-C₁₂)-alkylsulfinyl, phenylsulfonyl, phenylsulfinyl; where phenyl is optionally substituted once by fluorine, chlorine or (C₁-C₅)-alkoxy, (C₁-C₁₀)-alkoxy, -O-[CH₂]ₓ-C_{f}H_{(2f+i-g)}Fg or benzyloxy [lacuna] is optionally substituted once in the phenyl ring by fluorine, chlorine or (C₁-C₅)-alkoxy, including the physiologically active salts.

4. A compound of the formula I as claimed in claims 1 and 2, in which
Q is O,
X is O,
Y is CR³,
m is 0,
A is a -CH₂- group,
B is C0₂H,
R¹ is hydrogen, (C₁-C₁₀)-alkoxy, (C₅-C₆)-cycloalkyloxy, (C₅-C₆)-cycloalkyl-(C₁-C₂)-alkoxy, -O-[CH₂]ₓ C_{f}H_{(2f+1-g)}F_{g}, (C₁-C₄)-alkoxy-(C₁-C₄)-alkoxy, substituted phenoxy or substituted benzyloxy where the phenyl radical is substituted by a substituent from the group fluorine, chlorine, cyano, trifluoromethyl, (C₁-C₄)-alkyl or (C₁-C₄)-alkoxy, and
R² and R³ are hydrogen, including the physiologically active salts.

5. A compound of the formula I as claimed in claims 1 and 2, in which
Q is S,
X is O,
Y is C_{R}3,
m is 0,
A is a -CH₂- group,
B is -C0₂H,
R¹ is hydrogen, and
R² and R³ are hydrogen.

6. A compound of the formula I as claimed in claims 1 and 3, in which
Q is O,
X is O,
Y is C_{R}3,
m is 0,
A is a -CH₂- group,
B is -C0₂H,
R¹ is hydrogen, and
R² and R³, together with the pyridine carrying them, form a quinoline ring.

7. A compound of the formula I as claimed in claims 1 to 6, plus 3-hydroxypyridine-2-carboxylic acid N-(carboxymethyl)amide, N-(3-hydroxypicolinyl)-L-threonine and N-[(3-hydroxy-6-methoxy-2-quinolinyl)-carbonyl]-D-serine for use as a pharmaceutical.

8. A compound as claimed in claims 1 to 7 to be used for inhibiting collagen biosynthesis, including a compound of the formula I in which R⁵ is -CH₂-SH.

9. A compound as claimed in claims 1 to 7 as an inhibitor of prolyl hydroxylase, including a compound of the formula I in which R⁵ is -CH₂-SH.

10. A compound as claimed in claims 1 to 7 for use as a fibrosuppressive agent, including a compound of the formula I in which R⁵ is -CH₂-SH.

11. A compound as claimed in claims 1 to 7 for producing a pharmaceutical against fibrotic diseases, including a compound of the formula I in which R⁵ is -CH₂-SH.

12. A compound as claimed in claims 1 to 7 for producing a pharmaceutical against fibrotic diseases of the liver, including a compound of the formula I in which R⁵ is -CH₂-SH.

13. A compound as claimed in claims 1 to 7 for producing a pharmaceutical against fibrotic diseases of the lung, including a compound of the formula I in which R⁵ is -CH₂-SH.

14. A compound as claimed in claims 1 to 7 for producing a pharmaceutical against fibrotic diseases of the skin, including a compound of the formula I in which R⁵ is -CH₂-SH.

15. A prodrug of a compound of the formula I as claimed in claims 1 to 7.

16. A process for preparing compounds according to formula I as claimed in claims 1 to 7, in which formula A is a substituted alkylene moiety, B is C0₂H, Y is CR³ and m is 0 or 1, by
il.) reacting pyridine-2-carboxylic acids of the formula II (R11 is H) with the amino esters of the formula III to form the amide esters of the formula IV, or
i2.) reacting pyridine-2-carboxylic esters of the formula II (R¹¹ is lower alkyl); under the conditions of aminolysis, to form the compounds of the formula IV; 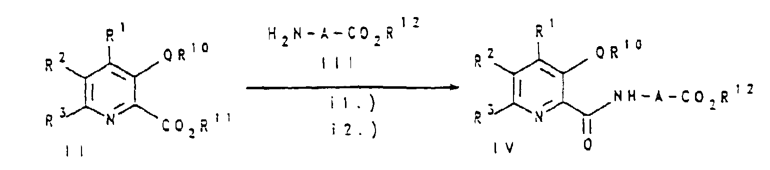 and
ii) liberating the compounds of the formulae I and V from their esters of the formula IV; 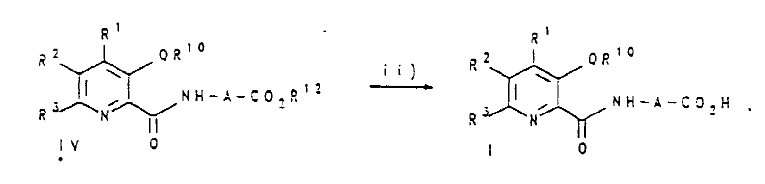 with, where appropriate,
iii) the compounds of the formula IV being prepared by alkylation of compounds of the formula V 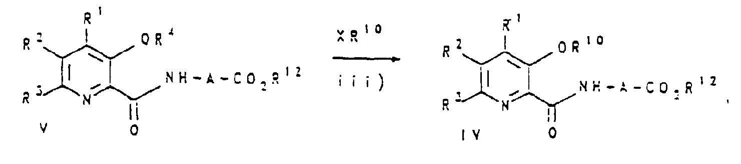 where X is a leaving group, in particular halogen, OS0₂Me or OS0₂ phenyl, and, where appropriate,
iv) the compounds of the formulae I, V or IV being converted into their pyridine N-oxides (I' or VI)

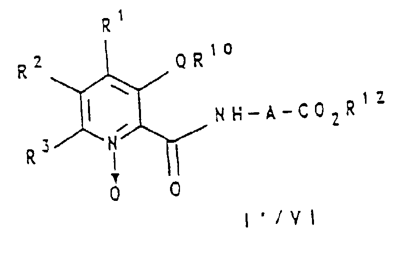

## Revendications

1. Composés de formule générale 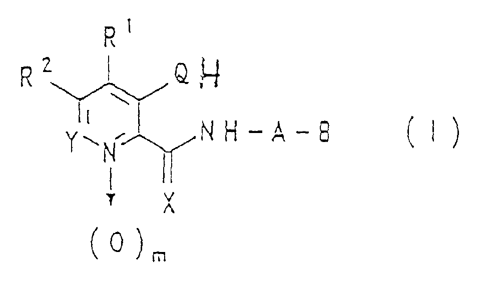 dans laquelle
Q représente un atome d'oxygène ou de soufre,
X représente un atome d'oxygène,
Y représente un groupe CR³,
m vaut 0 ou 1,
A représente un groupe alkylène en C₁-C₃ qui est éventuellement monosubstitué avec un atome d'halogène, un groupe cyano, trifluorométhyle, alkyle en C₁-C₅, hydroxyalkyle en C₁-C₅, alcoxy en C₁-C₅, -O[CH₂]ₓ-C_{f}H_{(2f+1-g)}F_{g} ou
A représente un groupe -CHR^{S-}, dans lequel R⁵ signifie l'un des substituant de l'atome de carbone situé en position a d'un a-aminoacide, en particulier d'un L-aminoacide naturel et de son isomère D, R⁵ ne représentant pas le groupe -CH₂-SH,
B représente le groupe C0₂H,
R² représente un atome d'hydrogène, un groupe alkyle en C₁-C₂₀, alkényle en C₂-C₂₀, alcynyle en C₂-C₂₀; alcoxy en C₁-C₂₀, alkényloxy en C₂-C₂₀, alcynyloxy en C₂-C₂₀, rétinyloxy, alcoxy en C₁-C₂₀-alkyle en C₁-C₃, alkényloxy en C₂-C₂₀-alkyle en C₁-C₃, rétinyloxy-alkyle en C₁-C₃, alcynyloxy en C₂-C₂₀-alkyle en C₁-C₃, un atome d'halogène, un groupe cyano, trifluorométhyle, hydroxyalkyle en C₁-C₈, alcanoyle en C₁-C₂₀, aralcanoyle en C₇-C₁₆; aroyle en C₆-C₁₂, aryle en C₆-C₁₂, aralkyle en C₇-C₁₆, -O[CH₂]ₓ-C_{f}H_{(2f+1-g)}F_{g}, NR'R", alkylmercapto en C₁-C₁₀, alkylsulfinyle en C₁-C₁₀, alkylsulfonyle en C₁-C₁₀, arylmercapto en C₆-C₁₂, arylsulfinyle en C₆-C₁₂, arylsulfonyle en C₆-C₁₂, aralkylmercapto en C₇-C₁₂, aralkylsulfinyle en C₇-C₁₂, aralkylsulfonyle en C₇-C₁₂, aryloxy en C₆-C₁₂, aralkyloxy en C₇-C₁₆, carboxy, alcoxy en C₁-C₂₀-carbonyle, alcoxy en C₁-C₁₂-alcoxy en C₁-C₁₂-carbonyle, aryloxy en C₆-C₁₂-carbonyle, aralcoxy en C₇-C₁₆-carbonyle, cycloalcoxy en C₃-C_{B}- carbonyle, alkényloxy en C₂-C₂₀-carbonyle, rétinyloxycarbonyle, alcynyloxy en C₂-C₂₀-carbonyle, cycloalkyle en C₃-C₈-alcoxy en C₁-C₆-carbonyle, cycloalkoxy en C₃-C₈-alcoxy en C₁-C₆₋carbonyle, aryloxy en C6-C₁₂ alcoxy en C₁-C₆₋carbonyle, aralcoxy en C₇-C₁₆-alcoxy en C₁-C₆-carbonyle, un groupe carbamoyle, N-alkyl en C₁-C₁₂-carbamoyle, N,N-dialkyl en C₁-C₁₂-carbamoyle, N-cycloalkyl en C₃-C₈-carbamoyle, N,N-dicycloalkyl en C₃ C₈ carbamoyle, N-alkyle en C₁-C_{1O}-N-cycloalkyl en C₃-C₈-carbamoyle, N-(cycloalkyle en C₃-C₈-alkyl en C₁-C₆)carbamoyle, N-alkyle en C₁-C₆-N-(cycloalkyl en C₃-C₈-alkyl en C₁-C₆)carbamoyle, N-(+)-déhydroabiétyl-carbamoyle, N-alkyle en C₁-C₆-N-(+)-déhydroabiétylcarbamoy- le, N-aryl en C₆-C₁₂-carbamoyle, N-aralkyl en C₇-C₁₆-carbamoyle, N-alkyle en C₁-C₁₀-N-aryl en C₆-C₁₆-carbamoyle, N-alkyle en C₁-C₁₀-N-aralkyl en C₇-C₁₆-carbamoyle, N-(alcoxy en C₁-C₁₂ alkyl en C₁-C₁₀)carbamoyle, N-(aryloxy en C₆-C₁₆-alkyl en C₁-C₁₀)carbamoyle; N-(aralkyloxy en C₇-C₁₆-alkyl en C1-C₁₀)carbamoyle, N-alkyle en C₁-C₁₀-N-(alcoxy en C₁-C₁₀-alkyl en C₁-C₁₀)carbamoyle, N-alkyle en C₁-C₁₀-N-(aryloxy en C₆-C₁₂-alkyl en C₁-C₁₀)carbamoyle, N-alkyle en C₁-C₁₀-N-(aralkyloxy en C₇-C₁₆-alkyl en C₁-C₁₀)carbamoyle, CON(CH₂)_{h;} formule dans laquelle un groupe CH₂ peut être remplacé par 0, S, N-alkylimino en C₁-Cₐ, N-cycloalkylimino en C₃-C₈, N-cycloalkyle en C₃-C₈-alkylimino en C₁-C₄, N-arylimino en C₆-C₁₂, N-aralk- ylimino en C₇-C₁₆ ou N-alcoxy en C₁-C₄-alkylimino en C₁-C₆.
le groupe aryle étant substitué comme défini pour R¹ et R³,
R¹ et R³ sont identiques ou différents et représentent un atome d'hydrogène, un atome d'halogène, un groupe alkyle en C₁-C₁₂, alcoxy en C₁-C₁₂, -O-[CH₂]x-C_{f}H_{(2f+1-g)}Hal_{g}, alcoxy en C₁-C₁₂₋alkyle en C₁-C_{12'} alcoxy en C₁-C₈-alcoxy en C₁-C_{12'} alcoxy en C₁-C₁₂-alcoxy en C₁-C₈-alkyle en C₂-C₆, aralkyloxy en C₇-C₁₁, cycloalkyle en C₃-C_{8;} cycloalkyle en C₃-C₈-alkyle en C₁-C₈, cycloalcoxy en C₃-C₈, cycloalkyle en C₃-C₈-alcoxy en C₁-C₈, cycloalcoxy en C₃-C₈-alkyle en C₁-C₈, cycloalkyloxy en C₃-C₈-alcoxy en C₁-C₈, cycloalkyle en C₃-C₈-alkyle en C₁-C₆-alcoxy en C₁-C₆, cycloalkyle en C₃-C₈-alcoxy en C₁-C₆-alkyle en C₁-C₆, cycloalcoxy en C₃-C₈-alcoxy en C₁-C₆-alkyle en C₁-C₆, NR^{Y}R^{z}; alkylmercapto en C₁-C₈, alkylsulfinyl en C₁-C₈ ou alkylsulfonyl en C₁-C₈, arylmercapto en C₆-C₁₂, arylsulfinyle en C₆-Cy₂, arylsulfonyle en C₆-Cy_{2;} aralkylmercapto en C₇-C₁₂, aralkylsulfinyle en C₇-C₁₁, aralkylsulfonyle en C₇-C₁₁, aryloxy substitué en C₆ C₁₂-alkyle en C₁-C₆, aralcoxy en C₇-C₁₁-alkyle en C₁-C₆, aryloxy en C₆-C₁₂ alcoxy en C₁-C₆-alkyle en C₁-C₆; aralkyloxy en C₇-C₁₁-alcoxy en C₁-C₆-alkyle en C₁-C₆, aryloxy en C₆-C₁₂, (C₇-C₁₁ )aralkyloxy, aryloxy en C₆-C₁₂-alcoxy en C₁-C₆ ou aralcoxy en C₇-C₁₁-alcoxy en C₁-C₆, un résidu aromatique portant 1, 2, 3, 4 ou 5 substituants identiques ou différents choisis parmi l'atome d'hydrogène, un atome d'halogène, le groupe cyano, nitro, trifluorométhyle, alkyle en C₁-C₆, alkényle en C₁-C₁₆, hydroxyalkyle en C₁-C₆, alcoxy en C₁-C₁₆, alkényloxy en C₁-C₁₆, -O[CH₂]x-C_{f}H(_{2f+1-g)}F_{g}: - OCF₂CI, -0-CF₂-CHFCI, alkylmercapto en C₁-C₆, alkylsulfinyle en C₁-C₆, arylsulfonyle en C₁-C₆, alkyl en C₁-C₆-carbonyle, alcoxy en C₁-C₆-carbonyle; carbamoyle, N-alkyl en C₁-C₄-carbamoyle, N,N-di-alkyl en C₁-C₄-carbamoyle, alkyl en C₁-C₆-carbonyloxy- cycloalkyl en C₃-C₈-carbamoyle, phényle, benzyle, phénoxy benzyloxy, NR^{Y}R^{Z}, phénylmercapto, phénylsulfonyle, phénylsulfinyle, sulfamoyle, N-alkylsulfamoyle en C₁-C₄ ou N,N-di-alkylsulfamoyle en C₁-C₄, ou portant éventuellement juqu'à 3 desdits précédents substituants identiques ou différents et deux atomes de carbone voisins du radical aralkyloxy portent ensemble une chaîne -[CH_{2]}- et/ou -CH=CH-CH=CH- dans laquelle un groupe CH₂ de la chaîne est éventuellement substitué par O, S, SO, SO₂ ou NR',
R² et R³ forment ensemble avec le noyau pyridine qui les porte un noyau quinoléine, de formule 1a, 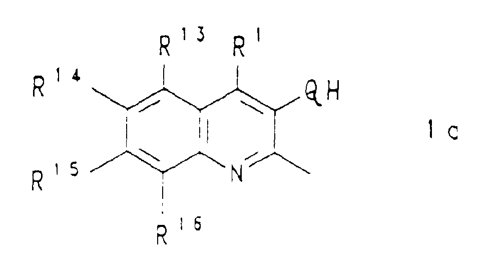 dans laquelle,
R13, R14, R¹⁵ et R¹⁶ représentent un atome d'hydrogène, un groupe alkyle en C₁-C₁₂, alkényle en C₁-C₁₂, un atome de chlore, fluor, brome, un groupe trifluorométhyle, alkylsulfonyle en C₁-C₁₂, alkylsulfinyle en C₁-C₁₂, phénylsulfonyle, phénylsulfinyle ; le groupe phényle étant éventuellement substitué par du fluor, du chlore ou un alcoxy en C₁-C₅, un groupe alcoxy en C₁-C₁₀,-O[CH₂]ₓ C_{f}H_{(2f+1-g})F_{g} ou un radical de formule D 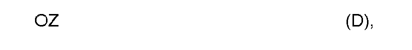 formule dans laquelle Z représente [CH₂]ᵥ-[O]_{w}-[CH₂]ₜ-E,
et où E représente un radical phényle substitué de formule F 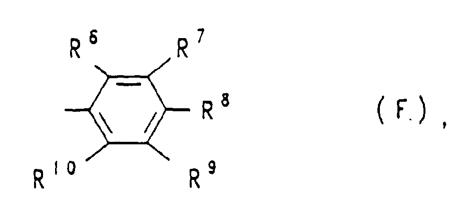
ou un radical hétéroaryle substitué ou un radical cycloalkyle substitué en C₃-C₈
où,
v = 0, 1, 2, 3, 4, 5, 6 w = 0, 1 et t = 0, 1, 2, 3, à condition que v soit différent de 0 quand w = 1, et où
R⁶, R⁷, R⁸; R⁹ et R¹⁰ sont identiques ou différents et représentent un atome d'hydrogène, d'halogène, un groupe cyano, nitro, trifluorométhyle, alkyle en C₁-C₆, cycloalkyle en C₃-C₈, alcoxy en C₁-C₆,-O[CH₂]ₓ-C_{f}H_{(2f+1-g)}F_{g}, - OCF₂CI, -0-CF₂-CHFCI, alkylmercapto en C₁-C₆; hydroxyalkyle en C₁-C₆, alcoxy en C₁-C₆-alcoxy en C₁-C₆, alcoxy en C₁-C₆-alkyle en C₁-C₆, alkylsulfinyle en C₁-C₆, alkylsulfonyle en C₁-C₆, alkyl en C₁-C₆-carbonyle, alcoxy en C₁-C₈-carbonyle, carbamoyle, N-alkyl en C₁-C₈-carbamoyle, N,N-di-alkyl en C₁-C₈-carbamoyle, aralkyl en C₇ C₁₁-carbamoyle éventuellement substitué avec des atomes de fluor, chlore, brome, des groupes trifluorométhyle et alcoxy en C₁-C₆, N-cycloalkyl en C₃-C₆-carbamoyle, N-cycloalkyle en C₃-C₈-alkyl en C₁-C₄-carbamoyle, alkyl en C₁-C₆-carbonyloxy, phényle, benzyle, phénoxy, benzyloxy, NR'R", tels que les groupes amino, anilino, N-mé- thylanilino, phénylmercapto, phénylsulfonyle, alcanoylimino en C₁-C₄ ou N-alcoxy en C₁-C₄-carbonylimino, et
f vaut de 1 à 8,
g vaut 0, 1 à (2f + 1 ),
h vaut de 3 à 6,
x vaut de 0 à 3, et
n est 3 ou 4,
y compris les sels physiologiquement actifs, à l'exception de la N-(3-hydroxy-picolinoyl)-L-thréonine, et de la N-[ (3-hydroxy-6-méthoxy-2-quinoléinyl)-carbonyl]-D-sérine.

2. Composés de formule I selon la revendication 1, dans laquelle
Q représente un atome d'oxygène ou de soufre,
X représente un atome d'oxygène,
Y représente un groupe CR³,
m vaut 0,
A représente un groupe alkylène en C₁-C₃,
B représente le groupe C0₂H,
R² représente un atome d'hydrogène, de brome, de chlore, un groupe cyano, un groupe alkyle en C₁-C₁₈, alcoxy en C₁-C₈, alcoxy en C₁-C₁₈-méthyle, alkényl en C₂-C₁₈-oxyméthyle, alcynyl en C₂-C₁₈-oxyméthyle, carbamoyle, N-alkyl en C₁-C₁₀-carbamoyle, N-(alcoxy en C₁-C₁₂-alkyl en C₁-C₄)carbamoyle, N,N-dialkyl en C₁-Cₐ-carbamoyle, N-cycloalkyl en C₃-C₈-carbamoyle, N-(C₆-C₁₂)-phénylcarbamoyle, N-phénylalkyl en C₇-C₁₂- carbamoyle, N-alkyle en C₁-C₆-N-phényl en C₆-C₁₂-carbamoyle, N-alkyle en C₁-C₆-N-phénylalkyl en C₇-C₁₂- carbamoyle, N-(alcoxy en C₁-C₆-alkyl en C₁-C₆)carbamoyle, carboxy, alcoxy en C₁-C²⁰-carbonyle, alkényl en C₂-C₂₀-oxycarbonyle, rétinyloxycarbonyle, cycloalcoxy en C₃-C₈-carbonyle, cycloalkyle en C₃-C₈-alcoxy en C₁-C₆-carbonyle, cycloalcoxy en C₃-C₈-alcoxy en C₁-C₆-carbonyle; phényle-alcoxy en C₁-C₆ carbonyle, phénoxy-alcoxy en C₁-C₆-carbonyle, benzyloxyalcoxy en C₁-C₆-carbonyle, un radical phényle étant substitué de la manière définie pour R¹ et R³ et l'un des radicaux
R¹ ou R³ représente un atome d'hydrogène et l'autre un résidu choisi parmi l'atome d'hydrogène, de fluor, de chlore, les groupes alkyle en C₁-C₈, alcoxy en C₁-C₁₀, cycloalkyle en C₅-C₆, cycloalkyle en C₅-C₆ alkyle en C₁-C₆, cycloalkyloxy en C₁-C₆, cycloalkyle en C₅-C₆-alcoxy en C₁-C₆, cycloalkyloxy en C₅-C₆-alkyle en C₁-C₆, cycloalkyloxy en C₅-C₆-alcoxy en C₁-C₆, cycloalkyle en C₅-C₆-alkyle en C₁-C₄- alcoxy en C₁-C₄, cycloalkyle en C₅-C₆-alcoxy en C₁-C₄-alkyle en C₁-C₂, cycloalcoxy en C₅-C₆-alcoxy en C₁-C₄-alkyle en C₁-C₂, -O[CH2]ₓC_{f}H_{(2f+1-g)}F_{g}, alcoxy en C₁-C₆-alkyle en C₁-C₆; alcoxy en C₁-C₆ alcoxy en C₁-C_{6;} alcoxy en C₁-C₆-alcoxy en C₁-C₄-alkyle en C₁-C₂, phénoxy substitué en C₆-C₁₂- phénylalkyloxy en C₇-C₁₁; phénoxy en C₆-C₁₂ alcoxy en C₁-C₆, ou phénylalkyloxy en C₇-C₁₁-alcoxy en C₁-C₆, phénoxyalkyle en C₁-C₄, phénylalkyloxy en C₇-C₁₁-alkyle en C₁-C₄, phénoxy-alcoxy en C₁-C₄-alkyle en C₁-C₂, phénylalkyloxy en C₇-C₁₁-alcoxy en C₁-C₄-alkyle en C₁-C₂, un radical aromatique étant substitué par 1, 2 ou 3 substituants identiques ou différents choisis parmi l'atome de fluor, de chlore, le groupe cyano, trifluorométhyle, alkyle en C₁-C₁₂, alkényle en C₂-C₁₂, alkényloxy en C₂-C₁₂, alcoxy en C₁-C₁₂.
au cas où R¹ ou R³ représente un groupe phénoxy en C₆-C₁₂, phénylalkyloxy en C₇-C₁₁, phénoxy en C₆-C₁₂ alcoxy en C₁-C₆, phényalcoxy en C₇-C₁₁-alcoxy en C₁-C₆,
cycloalkyloxy en C₅-C₆,
cycloalkyle en C₅-C₆-alcoxy en C₁-C₆, cycloalcoxy en C₅-C₆-alcoxy en C₁-C₆ ou cycloalkyle en C₅-C₆-alkyle C₁-C₄-alcoxy en C₁-C₄, ce radical est en particulier un radical de formule D 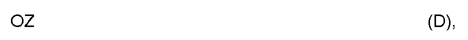 formule dans laquelle Z représente [CH2]ᵥ-[O]_{w}-[CH₂]ₜ-E,
et dans laquelle E représente un radical phényle substitué de formule F 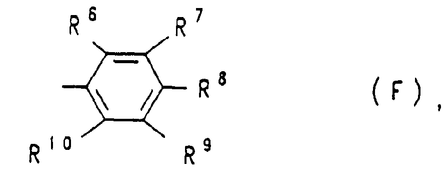 ou un radical cycloalkyle en C₃-C₈, où,
v peut être 0, 1, 2, 3, w 0, 1 et t = 0, 1, à condition que v soit différent de 0 quand w = 1, et dans laquelle R⁶, R⁷, R⁸; R⁹ et R¹⁰ sont identiques ou différents et représentent un atome d'hydrogène, de fluor, de chlore, un groupe cyano, trifluorométhyle, alkyle en C₁-C₆, alcoxy en C₁-C₆, -O[CH₂]ₓ-C_{f}H_{(2f+1-g})F_{g}, N-alkyl en C₁-C₈-carbamoyle, N,N-di-alkyl en C₁-C₈-carbamoyle, N-cycloalkyl en C₃-C₈-carbamoyle, phénylalkyl en C₇-C₁₁ -carbamoyle éventuellement substitué par des atomes de fluor, chlore, des groupes trifluorométhyle et alcoxy en C₁-C₆, ou
au cas où R¹ ou R³ représente un groupe phényle, phénoxy-alkyle en C₁-C₆, phénylalkyle en C₇-C₁₁, phénylalcoxy en C₇-C₁₁-alkyle en C₁-C₄, cycloalkyle en C₅-C₆, cycloalkyle en C₅-C₆-alkyle en C₁-C₆, cycloalcoxy en C₅-C₆ alkyle en C₁-C₄, cycloalkyle en C₅-C₆-alcoxy en C₁-C₄-alkyle en C₁-C₂, cycloalcoxy en C₅-C₆-alcoxy en C₁-C₄ alkyle en C₁-C₂, ce radical est en particulier un radical de formule D,
dans laquelle
v sont égaux à 1, 2, 3 et 4, w = et t = 0 ou
v sont égaux à 1, 2, 3 et 4, w = 1 et t = 0 ou
v sont égaux à 1, 2, 3 et 4, w = et t = 1.

3. Composés de formule I la revendication 1, dans laquelle
Q représente un atome d'oxygène, de soufre, de préférence un atome d'oxygène,
X représente un atome d'oxygène,
Y représente un groupe CR³,
m vaut 0,
A représente un groupe -CH₂-,
B représente le groupe C0₂H,
R¹ représente un atome d'hydrogène
R² et R³ forment ensemble avec le noyau pyridine les portant un cycle quinoléine,
caractérisé en ce que
R¹³, R¹⁵ et R¹⁶ représentent un atome d'hydrogène, R¹⁴ représente un atome d'hydrogène, un groupe alkyle en C¹-C¹², alkényle en C¹-C¹², un atome de chlore, fluor, brome, un groupe trifluorométhyle, alkylsulfonyle en C¹-C¹², alkylsulfinyle en C¹-C¹2, phénylsulfonyle, phénylsulfinyle ; le groupe phényle étant éventuellement monosubstitué par un atome de fluor, de chlore ou un alcoxy en C₁-C₅, alcoxy en C₁-C_{10;} -O[CH₂]ₓ C_{f}H_{(2f+1-g)}F_{g}, benzyloxy, le noyau phényle étant éventuellement monosubstitué par un atome de fluor, de chlore ou un groupe alcoxy en C₁-C₅, y compris leurs sels physiologiquement actifs.

4. Composés de formule I selon les revendications 1 et 2, dans laquelle
Q représente un atome d'oxygène,
X représente un atome d'oxygène,
Y représente un groupe CR³,
m vaut 0,
A représente un groupe -CH₂ ,
B représente le groupe C0₂H,
R¹ représente un atome d'hydrogène, un groupe alcoxy en C₁-C_{10;} cycloalkyloxy en C₅-C₆, cycloalkyle en C₅-C₆-alcoxy en C₁-C₂, -O[CH₂]ₓ C_{f}H_{(2f+1-g)}F_{g}, alcoxy en C₁-C₄-alcoxy en C₁-C₄, phénoxy substitué, benzyloxy substitué, le radical phényle étant substitué par un substituant choisi parmi l'atome de fluor, de chlore, le groupe cyano, trifluoro-méthyle, alkyle en C₁-C₄ ou alcoxy en C₁-C₄ et
R² et R³ représentent un atome d'hydrogène, y compris leurs sels physiologiquement actifs.

5. Composés de formule I selon les revendications 1 et 2, dans laquelle
Q représente un atome de soufre,
X représente un atome d'oxygène,
Y représente un groupe CR³,
m vaut 0,
A représente un groupe -CH₂-,
B représente le groupe C0₂H,
R¹ représente un atome d'hydrogène,
R² et R³ représentent un atome d'hydrogène.

6. Composés de formule I selon les revendications 1 et 3, dans laquelle
Q représente un atome d'oxygène,
X représente un atome d'oxygène,
Y représente un groupe CR³,
m vaut 0,
A représente un groupe -CH₂-,
B représente le groupe C0₂H,
R¹ représente un atome d'hydrogène,
R² et R³ forment ensemble avec le noyau pyridine les portant un noyau quinoléine.

7. Composés deformule 1 selon les revendications 1 à plus le N-(carboxyméthyl)amide de l'acide 3-hydroxypyridine-2-carboxylique, la N-(3-hydroxypicolinyl)-L-thréonine et la N-[(3-hydroxy-6-méthoxy-2-quinoléinyl)-carbonyl]-D-sérine pour utilisation comme médicament.

8. Composés selon les revendications 1 à 7 pour application à l'inhibition de la biosynthèse du collagène, y compris les composés de formule I; dans lesquels R⁵ représente -CH₂-SH.

9. Composés selon les revendications 1 à 7 comme inhibiteurs de la prolylhydrolase, y compris les composés de formule 1, dans lesquels R⁵ représente -CH₂-SH.

10. Composés selon les revendications 1 à 7 pour application comme fibrosuppresseur, y compris les composés de formule 1, dans lesquels R⁵ représente -CH₂-SH.

11. Composés selon les revendications 1 à 7 pour la fabrication d'un médicament contre les maladies fibreuses, y compris les composés de formule I, dans lesquels R⁵ représente -CH₂-SH.

12. Composés selon les revendications 1 à 7 pour la fabrication d'un médicament contre les maladies fibreuses du foie, y compris les composés de formule I, dans lesquels R⁵ représente -CH₂-SH.

13. Composés selon les revendications 1 à 7 pour la fabrication d'un médicament contre les maladies fibreuses du poumon, y compris les composés de formule I, dans lesquels R⁵ représente -CH₂-SH.

14. Composés selon les revendications 1 à 7 pour la fabrication d'un médicament contre les maladies fibreuses de la peau, y compris les composés de formule I, dans lesquels R⁵ représente -CH₂-SH.

15. Promédicaments des composés de formule I selon les revendications 1 à 7.

16. Procédé de fabrication de composés conformes à la formule I selon les revendications 1 à 7, dans lequel A représente une partie alkyle substituée, B = CO₂H, Y = CR³ et m = et 1, caractérisé en ce que
i1. on fait réagir des acides pyridin-2-carboxyliques de formule II (R¹¹ = H) avec les aminoesters de formule III pour obtenir les composés ester-amide de formule IV, ou
i2. on fait réagir des esters des acides pyridin-2-carboxyliques de formule II (R¹¹ = alkyle inférieur) dans les conditions de l'aminolyse pour obtenir les composés de formule IV : 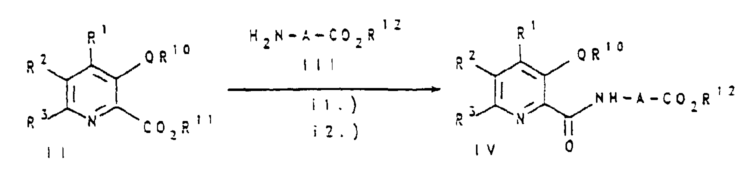 et
ii) on libère les composés de formules I et V à partir de leurs esters de formule IV : 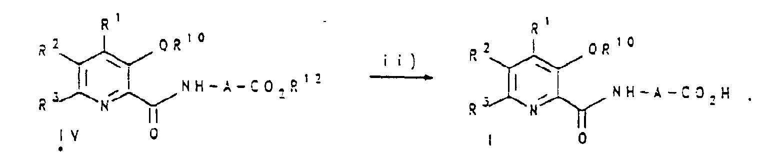 et éventuellement caractérisé en ce que
iii) on fabrique les composés de formule IV par alkylation de composés de formule V, 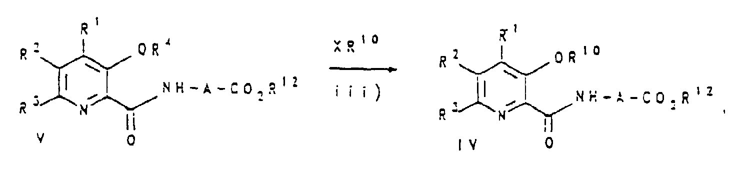 où X représente un groupe partant, en particulier un atome d'halogène, un groupe OS0₂Me, OS0₂phényl et éventuellement
iv) on transforme les composés de formules I, V ou IV en leur dérivé N-oxyde-pyridine (I' ou VI).
